# FASCICULE DE BREVET EUROPEEN

(11) **EP 0 567 577 B1**
(45) Date de publication et mention de la délivrance du brevet: **21.08.2002**
(21) Numéro de dépôt: 92904705.8
(22) Date de dépôt: 14.01.1992
(51) Int. Cl.: C12N 15/12, C07K 14/00, C12P 21/08, G01N 33/50, A01K 67/027

(54) **SEQUENCES NUCLEOTIDIQUES CODANT POUR LE RECEPTEUR BETA 3 ADRENERGIQUE MURIN ET LEURS APPLICATIONS**
NUKLEOTIDE SEQUENZEN DIE FÜR DEN MURIN-BETA-3-ADRENERGISCHEN REZEPTOR KODIEREN UND IHRE VERWENDUNGEN
NUCLEOTIDE SEQUENCES CODING FOR THE MURINE ADRENERGENIC BETA 3 RECEPTOR AND APPLICATIONS THEREOF

(30) Priorité: 14.01.1991 FR 9100320
(43) Date de publication de la demande: 03.11.1993
(73) Titulaire: CENTRE NATIONAL DE LA RECHERCHE SCIENTIFIQUE, 75016 Paris Cédex (FR)
(72) Inventeur: NAHMIAS, Clara, F-75004 Paris (FR); EMORINE, Laurent, Jean, F-75013 Paris (FR); STROSBERG, Arthur, Donny, F-75015 Paris (FR)
(74) Mandataire: Ores, Irène
(86) Numéro de dépôt international: FR9200023
(87) Numéro de publication internationale: WO92012246

(56) Documents cités:
- EP-A- 0 351 921
- WO-A-90/08775
- EMBO JOURNAL. vol. 10, no. 12, Décembre 1991, EYNSHAM, OXFORD GB pages 3721 - 3727; Nahmias C;Blin N;Elalouf JM;Mattei MG;Strosberg AD;Emorine LJ: 'Molecular characterization of the mouse beta 3-adrenergic receptor: relationship with the atypical receptor of adipocytes.'
- SCIENCE. vol. 245, 8 Septembre 1989, LANCASTER, PA US pages 118 - 1121; Emorine, L.J. et al.: 'Molecular characterization of the human â3-adrenergic receptor.'

## Description

La présente invention est relative à des séquences nucléotidiques codant pour le récepteur β3-adrénergique (RAβ3) murin, à l'utilisation desdites séquences comme sondes et pour l'expression de peptides et/ou de fragments de ceux-ci ayant une activité de RAβ3 murin, aux vecteurs utiles pour ladite expression, ainsi qu'aux hôtes cellulaires contenant ledit vecteur.

La présente invention est également relative à des anticorps polyclonaux et monoclonaux dirigés contre lesdits peptides et utilisables, notamment pour la détection de récepteurs β3-adrénergiques murins, ainsi qu'à un procédé de criblage de substances, à action agoniste ou antagoniste vis-à-vis des peptides ayant une action de récepteur β3-adrénergique et à des trousses ou kits pour la détection du degré d'affinité de différentes substances pour lesdits peptides à activité de récepteur β3-adrénergique. De telles substances sont notamment utilisables comme médicaments, dans le traitement de l'obésité, du diabète gras et du diabète des sujets non insulino-dépendants ainsi que dans le traitement des hyperlipidémies.

Il est connu que les catécholamines telles que l'adrénaline et la noradrénaline, les agonistes synthétiques de ces catécholamines, qui miment leurs fonctions biologiques et les antagonistes, qui bloquent ces fonctions biologiques, exercent leurs effets en se liant à des sites de reconnaissance (récepteurs membranaires) spécifiques, situés sur les membranes cellulaires.

Deux classes principales de récepteurs adrénergiques ont été définies, les récepteurs adrénergiques α et les récepteurs adrénergiques β.

Dans l'ensemble de ces deux classes, on distingue, maintenant, cinq sous-types de récepteurs aux catécholamines (α1, α2, β1, β2 et β3-RA). Leurs gènes ont été récemment isolés et identifiés (S. COTECCHIA et al., 1988, Proc. Natl. Acad. Sci. USA, 85, 7159-7163 ; B.K. KOBILKA et al., 1987, Science, 238, 650-656 ; T. FRIELLE et al., 1987, Proc. Natl. Acad. Sci. USA 84, 7920-7924 ; L.J. EMORINE et al., 1987, Proc. Natl. Acad. Sci., USA, 84, 6995-6999 ; L.J. EMORINE et al., 1989, Science, 245, 1118-1121). L'analyse de ces gènes a permis de reconnaître leur appartenance à une famille de récepteurs membranaires intégraux présentant certaines homologies (R.A.F. DIXON et al., 1998, Annual Reports in Médicinal Chemistry, 221-233 ; L.J. EMORINE et al., 1988, Proc. NATO Adv. Res. Workshop), notamment au niveau de 7 régions transmembranaires, qui sont couplées à des protéines régulatrices, appelées protéines G, susceptibles de fixer des molécules de guanosine triphosphate (GTP).

Ces récepteurs membranaires, lorsqu'ils ont fixé le ligand approprié (agoniste ou antagoniste), subissent un changement de conformation, qui induit un signal intracellulaire, qui modifie le comportement de la cellule cible.

Dans le cas des récepteurs β-adrénergiques, lorsqu'ils se lient avec des agonistes des catécholamines, ils catalysent l'activation d'une classe de protéines G, qui stimule à son tour l'activité de l'adénylate cyclase, alors que les antagonistes des RAβ agissent en compétition avec les agonistes pour la liaison au récepteur et empêchent l'activation de l'adénylate cyclase.

Lorsque l'adénylate cyclase est activée, elle catalyse la production d'un médiateur intracellulaire ou second messager, notamment l'AMP cyclique.

Les Inventeurs ont récemment mis en évidence de nouveaux récepteurs β-adrénergique chez l'Homme, dénommés RA-Huβ3, et caractérisés par des propriétés différentes de celles des récepteurs β1 et β2, notamment en ce qu'ils se comportent de façon différente vis-à-vis de substances respectivement antagonistes et agonistes des récepteurs β1 et β2 (Demande de Brevet français n° 89 00918).

Un tel récepteur RA-Huβ3 est plus particulièrement constitué par une séquence de 402 amino-acides et est considéré comme comportant également sept régions transmembranaires hydrophobes séparées par des boucles hydrophiles intra- et extra-cellulaires.

Poursuivant leurs travaux dans cette voie, les Inventeurs, ont cherché à mettre en évidence un tel PAβ3 chez les rongeurs et plus particulièrement chez les murins afin de pouvoir disposer notamment d'un modèle d'étude des ligands β-adrénergiques.

La présente invention a pour objet une séquence d'acide nucléique, caractérisée en ce qu'elle comprend le gène murin codant pour un peptide ayant une activité de récepteur β3-adrénergique, c'est-à-dire qu'il est capable de participer à l'activation de l'adénylate cyclase en présence d'au moins un des agonistes suivants : BRL 28410, BRL 37344, CGP 12177A et (l)-isoprotérénol, en ce qu'elle comporte 1920 paires de bases et comprend la séquence en nucléotides de formule I ci-après :

GATCTGTAATCCCAGCACTGGGGAGGTTGAGGCAGAAGGATCTGGAGGTCCAGACCAATCTGGGCAACATATAGAAAGACTATCTCAAACAATAAGATAC 100

CTTAGGGAGAGCATCCAAGCAGAAGAGGGGCTATCTTGGATGGTTTGGGTTGTTCGGTTTTGTTTTGGTTTGTTTCTGGATGGTTGCCTTCCTTGTTGGG 200

TAAAGGATAGGGTGCGGGGGTTTCTCTTCTTTGCAGGGTTGCCTCAGGTTCTGCCAGGAAGGAGCTGCTGAGCTCCAGGAAACCGGTGCTGAGGGAGTGT 300

CAAGACAGGACGCCCCTCTCCACCCTCCAATTCCCACCAGAGGCCTCTCTTGTGACTATTGGACGCTGTTCCTTTAAAAGCAGCCACTCCTCCCGGCAAC 400

TAGGGTGTACATGGGGGGTGAGATGGAGGGAAGCTGACAGACTTACCCCAGCAATTAGGGAAGATGGCCCAGGCTGGAAGAGTCGCTCCCAAGCCCTACT 500

GTCCCCTTCCCTAAGCCAGCGGGTCTGGGGAGGAGGGGAACCTTCCCACCCCAGGCGCCACACGAG ATG GCT CCG TGG CCT CAC AGA AAC 591

GGC TCT CTG GCT TTG TGG TCG GAC GCC CCT ACC CTG GAC CCC AGT GCA GCC AAC ACC AGT GGG TTG CCA GGA GTA 666

TM1
CCA TGG GCA GCG GCA TTG GCT GGG GCA TTG CTG GCG CTG GCC ACG GTG GGA GGC AAC CTG CTG GTA ATC ATA GCC 741

TM1 ↵ TM2
ATC GCC CGC ACG CCG AGA CTA CAG ACC ATA ACC AAC GTG TTC GTG ACT TCA CTG GCC GCA GCT GAC TTG GTA GTG 816

TM2 ↵
GGA CTC CTC GTA ATG CCA CCA GGG GCC ACA TTG GCG CTG ACT GGC CAT TGG CCC TTG GGC GAA ACT GGT TGC GAA 891

→TM3 TM3 ↵
CTG TGG ACG TCA GTG GAC GTG CTC TGT GTA ACT GCT AGC ATC GAG ACC TTG TGC GCC CTG GCT GTG GAC CGC TAC 966

TM4
CTA GCT GTC ACC AAC CCT TTG CGT TAC GGC ACG CTG GTT ACC AAG CGC CGC GCC CGC GCG GCA GTT GTC CTG GTG 1041

TM4 ↵
TGG ATC GTG TCC GCT GCC GTG TCC TTT GCG CCC ATC ATG AGC CAG TGG TCC CGT GTA GGG GCA GAT GCC GAG GCA 1116

→TM5
CAG GAA TGC CAC TCC AAT CCG CGC TGC TGT TCC TTT GCC TCC AAC ATG CCC TAT GCG CTC CTC TCC TCC TCC GTC 1191

TM5 ↵
TCC TTC TAC CTT CCC CTC CTT GTG ATG CTC TTC GTC TAT GCT CGA GTG TTC GTT GTG GCT AAG CGC CAA CGG CAT 1266

TTG CTG CGC CGG GAA CTG GGC CGC TTC TCG CCC GAG GAG TCT CCG CCG TCT CCG TCG CGC TCT CCG TCC CCT GCC 1341

ACA GGC GGG ACA CCC GCG GCA CCG GAT GGA GTG CCC CCC TGC GGC CGG CGG CCT GCG CGC CTC CTG CCA CTC CGG 1416

→TM6
GAA CAC CGC GCC CTG CGC ACC TTA GGT CTC ATT ATG GGC ATC TTC TCT CTG TGC TGG CTG CCC TTC TTC CTG GCC 1491

TM6 ↵ →TM7
AAC GTG CTG CGC GCA CTC GCG GGG CCC TCT CTA GTT CCC AGC GGA GTT TTC ATC GCC CTG AAC TGG CTG GGC TAT 1566

TM7 ↵
GCC AAC TCC GCC TTC AAC CCG GTC ATC TAC TGC CGC AGC CCG GAC TTT CGC GAC GCC TTC CGT CGT CTT CTG TGT 1641

AGC TAC GGT GGC CGT GGA CCG GAG GAG CCA CGC GCA GTC ACC TTC CCA GCC AGC CCT GTT GAA GCC AGG CAG AGT 1716

CCA CCG CTC AAC AGG TAG GGGACACGAGCGGGGGACCGGAGTCTCTGGGTGGGGACGTCTCTGTCTCTATTTTTGAGTTTGGAGATTGGGGAG 1810

GGGAAGATGTAGATGGGGGTGCGGTGTGTGTGTGGGTGGGGGGTGGCCTTTGTCTTGAGAGGACAGAAAAGAGGTAGGAACTAAAACGGGCCCTTTCTCT 1910

TCTTGGATCC (I) 1920

L'agoniste CGP 12177A est notamment décrit dans l'article au nom de N. MOHELL et al. (Biochem. J., 1989, 261, 401-405) et les agonistes BRL 28410 et BRL 37344 sont décrits dans l'article au nom de J.R.S. ARCH et al. (Nature, 1984, 309, 5964, 163-165).

Ladite séquence comprend notamment les sites de restriction uniques suivants :
AlwN I, Sac I, Stu I, Dra I, Mse I, Bbe I, Nar I, Xcm I, NCo I, BspM I, Afl III, Pvu I, Nhe I, BstE II, BspH I, Bsm I, Nsp7524 I, NspH I, Xho I, Sac II, Eag I, Nae I, PaeR7 I, EcoN I, Fsp I, Bbv II, Nru I, Drd I, BamH I.

L'invention a également pour objet les fragments de ladite séquence utiles pour l'expression du peptide correspondant et/ou la détection du gène murin codant pour le récepteur β3 murin.

Parmi lesdits fragments, elle englobe entre autres :
- une séquence constituée d'un segment de 1164 paires de bases nucléotidiques, qui correspond aux nucléotides 568-1731 de la séquence de formule I.
- une séquence constituée d'un segment de 1360 paires de bases contenant la région codante du gène β3 murin et une partie de la région 3' non traduite.
- une séquence constituée d'un segment de 280 paires de bases nucléotidiques, qui correspond aux nucléotides 1640-1920 de la séquence de formule I.
- une séquence constituée d'un segment de 608 paires de bases nucléotidiques, situé entre les régions transmembranaires TM3 et TM6 et qui correspond aux nucléotides 895-1503 de la séquence de formule I.
- une séquence constituée d'un segment de 297 paires de bases nucléotidiques, allant du codon d'initiation de la traduction jusqu'à la fin du domaine TM2 et qui correspond aux nucléotides 567-864 (région du gène codant pour la partie amino-terminale du peptide) de la séquence de formule I.

Font également partie de l'invention, les acides nucléiques variants par rapport à ceux ci-dessus définis et qui comportent certaines mutations localisées dans la mesure où ces acides nucléiques variants s'hybrident avec les acides nucléiques précédemment définis ou avec des fragments de ceux-ci.

On entend, au sens de la présente invention, par séquence dérivée, aussi bien une séquence d'ADN ou d'ARN simple-brin ou double-brin, que leurs séquences complémentaires.

La présente invention a également pour objet des sondes nucléotidiques, caractérisées en ce qu'elles comprennent une séquence conforme à l'invention et en ce qu'elles sont éventuellement marquées à l'aide d'un marqueur tel qu'un isotope radioactif, une enzyme appropriée ou toute autre substance non radioactive appropriée.

Les conditions d'hybridation sont définies comme suit :

Pour les sondes les plus courtes, c'est-à-dire d'environ 10 à environ 100 nucléotides, des conditions d'hybridation appropriées sont les suivantes :
750 mM de NaCl, 75 mM de Tri-sodium citrate, 50 µg/ml d'ADN de sperme de saumon, 50 mM de phosphate de sodium, 1 mM de pyrophosphate de sodium, 100 µM d'ATP, 10 à 25 % de formamide, 1 % Ficoll ("PHARMACIA" poids moléculaire moyen de 400,00), 1 % de polyvinylpyrrolidone, 1 % de sérum albumine bovine, pendant 14 à 16 h à 42°C.

Pour les sondes les plus longues, c'est-à-dire présentant plus d'environ 100 nucléotides, des conditions d'hybridation appropriées sont celles indiquées précédemment pour les sondes les plus courtes, mais dans lesquelles le milieu sus-défini contient 40 % de formamide au lieu de 10 à 25 % de formamide.

Selon une disposition avantageuse de ce mode de réalisation, ladite sonde peut être avantageusement définie par l'une quelconque des séquences nucléotidiques définies ci-dessus et notamment :
- le fragment de 1920 paires de base, qui correspond à la totalité de la séquence de formule I
- le fragment d'environ 600 pb, qui correspond au fragment situé entre les régions transmembranaires TM3 et TM6 de la séquence de formule I ci-dessus ;
- le fragment d'environ 300 pb qui correspond à la région du gène codant pour la partie amino-terminale du peptide, allant du codon d'initiation de la traduction jusqu'à la fin du domaine TM2.
- le fragment d'environ 280 paires de bases qui correspond au segment 1640-1920 de la séquence de formule I.

De telles sondes ont notamment l'avantage de permettre la caractérisation des lignées consanguines génétiquement définies de souris diabétiques et/ou obèses, notamment des lignées db/db, ob/ob, NOD.

La présente invention a également pour objet un peptide et/ou un fragment de peptide, caractérisé en ce qu'il est codé par une séquence nucléotidique telle que définie ci-dessus et en ce qu'il présente une activité de récepteur β3-adrénergique.

Une activité de récepteur β3-adrénergique est celle définie dans la Demande de Brevet français n° 89 00918, à savoir que, lorsque le fragment est exposé à la surface d'une cellule, il est capable de participer à l'activation de l'adénylate cyclase en présence d'un des agonistes suivants : BRL 28410, BRL 37344, CGP 12177A et (1)-isoprotérénol ; soit il est susceptible d'être reconnu par des anticorps qui ne reconnaissent ni le récepteur adrénergique β1, ni le récepteur adrénergique β2 ; soit il est susceptible de générer des anticorps qui ne reconnaissent ni le récepteur β1, ni le récepteur β2.

Selon un mode de réalisation avantageux dudit peptide, il comprend 388 amino-acides et présente la séquence en amino-acides de formule II suivante : 
Met-Ala-Pro-Trp-Pro-His-Arg-Asn-Gly-Ser-Leu-Ala-Leu-Trp-
Ser-Asp-Ala-Pro-Thr-Leu-Asp-Pro-Ser-Ala-Ala-Asn-Thr-Ser-
Gly-Leu-Pro-Gly-Val-Pro-Trp-Ala-Ala-Ala-Leu-Ala-Gly-Ala-
Leu-Leu-Ala-Leu-Ala-Thr-Val-Gly-Gly-Asn-Leu-Leu-Val-Ile-
Ile-Ala-Ile-Ala-Arg-Thr-Pro-Arg-Leu-Gln-Thr-Ile-Thr-Asn-
Val-Phe-Val-Thr-Ser-Leu-Ala-Ala-Ala-Asp-Leu-Val-Val-Gly-
Leu-Leu-Val-Met-Pro-Pro-Gly-Ala-Thr-Leu-Ala-Leu-Thr-Gly-
His-Trp-Pro-Leu-Gly-Glu-Thr-Gly-Cys-Glu-Leu-Trp-Thr-Ser-
Val-Asp-Val-Leu-Cys-Val-Thr-Ala-Ser-Ile-Glu-Thr-Leu-Cys-
Ala-Leu-Ala-Val-Asp-Arg-Tyr-Leu-Ala-Val-Thr-Asn-Pro-Leu-
Arg-Tyr-Gly-Thr-Leu-Val-Thr-Lys-Arg-Arg-Ala-Arg-Ala-Ala-
Val-Val-Leu-Val-Trp-Ile-Val-Ser-Ala-Ala-Val-Ser-Phe-Ala-
Pro-Ile-Met-Ser-Gln-Trp-Trp-Arg-Val-Gly-Ala-Asp-Ala-Glu-
Ala-Gln-Glu-Cys-His-Ser-Asn-Pro-Arg-Cys-Cys-Ser-Phe-Ala-
 
Ser-Asn-Met-Pro-Tyr-Ala-Leu-Leu-Ser-Ser-Ser-Val-Ser-Phe-
Tyr-Leu-Pro-Leu-Leu-Val-Met-Leu-Phe-Val-Tyr-Ala-Arg-Val-
Phe-Val-Val-Ala-Lys-Arg-Gln-Arg-His-Leu-Leu-Arg-Arg-Glu-
Leu-GLy-Arg-Phe-Ser-Pro-Glu-Glu-Ser-Pro-Pro-Ser-Pro-Ser-
Arg-Ser-Pro-Ser-Pro-Ala-Thr-Gly-Gly-Thr-Pro-Ala-Ala-Pro-
Asp-Gly-Val-Pro-Pro-Cys-Gly-Arg-Arg-Pro-Ala-Arg-Leu-Leu-
Pro-Leu-Arg-Glu-His-Arg-Ala-Leu-Arg-Thr-Leu-Gly-Leu-Ile-
Met-Gly-Ile-Phe-Ser-Leu-Cys-Trp-Leu-Pro-Phe-Phe-Leu-Ala-
Asn-Val-Leu-Arg-Ala-Leu-Ala-Gly-Pro-Ser-Leu-Val-Pro-Ser-
Gly-Val-Phe-Ile-Ala-Leu-Asn-Trp-Leu-Gly-Tyr-Ala-Asn-Ser-
Ala-Phe-Asn-Pro-Val-Ile-Tyr-Cys-Arg-Ser-Pro-Asp-Phe-Arg-
Asp-Ala-Phe-Arg-Arg-Leu-Leu-Cys-Ser-Tyr-Gly-Gly-Arg-Gly-
Pro-Glu-Glu-Pro-Arg-Ala-Val-Thr-Phe-Pro-Ala-Ser-Pro-Val-
Glu-Ala-Arg-Gln-Ser-Pro-Pro-Leu-Asn-Arg (II),
 lequel peptide présente 82 % d'homologie avec le peptide β3 humain (récepteur β3 humain) .

Ce peptide est dénommé ci-après récepteur β3-adrénergique murin (RA-Muβ3).

Il n'était pas évident que les murins possédassent un récepteur β3-adrénergique et que ce dernier soit, de plus, suffisamment homologue au récepteur β3-adrénergique humain, pour permettre son utilisation comme modèle d'étude générale du récepteur β3-adrénergique.

Le RA-β3 murin (RA-Muβ3) présente, en effet, un certain nombre d'avantages :
- il est particulièrement bien adapté en tant que modèle du récepteur β3-adrénergique, en particulier pour des études physiologiques, qui ne peuvent pas être réalisées avec le récepteur humain ;
- il permet d'étudier la régulation de l'expression du récepteur β3-adrénergique, dans différentes conditions physiologiques (exposition au froid ou à des ligands β-adrénergiques), qui peuvent modifier l'activité lipolytique et/ou thermogénique, car le récepteur β3-adrénergique intervient dans les maladies telles que le diabète et/ou l'obésité, dans la mesure où il est exprimé dans des tissus qui jouent un rôle important dans le métabolisme (tissus adipeux, muscle squelettique notamment) ;
- il permet en outre d'analyser l'expression du récepteur β3-adrénergique au cours du développement embryonnaire.

L'invention comprend également les peptides variants de ceux définis ci-dessus, qui comportent certaines mutations, sans que les peptides ne perdent les propriétés de récepteur β₃-adrénergique. Parmi ces variants, on peut mentionner ceux qui sont reconnus par des anticorps reconnaissant les régions transmembranaires, ainsi que ceux qui sont reconnus par des anticorps reconnaissant les régions autres que les régions transmembranaires.

La présente invention a également pour objet des fragments ou des combinaisons de fragments du RA-Muβ3, conforme à l'invention et notamment :
- un fragment de 25 amino-acides, correspondant à la région transmembranaire TMI de la séquence de formule II ;
- un fragment de 30 aminoacides, correspondant au fragment COOH terminal de la séquence de formule II.

Lesdits fragments sont avantageusement obtenus par synthèse, notamment par la méthode de Merrifield.

La présente invention a également pour objet un vecteur recombinant de clonage et/ou d'expression, caractérisé en ce qu'il comprend une séquence nucléotidique conforme à l'invention.

On entend au sens de la présente invention, par vecteur recombinant, aussi bien un plasmide, un cosmide qu'un phage.

Selon un mode de réalisation avantageux dudit vecteur, il est constitué par un vecteur recombinant approprié comprenant en particulier une origine de réplication dans un microorganisme hôte approprié, notamment une bactérie ou une cellule eucaryote, au moins un gène dont l'expression permet la sélection soit des bactéries, soit des cellules eucaryotes ayant reçu ledit vecteur, une séquence régulatrice appropriée, notamment un promoteur permettant l'expression des gènes dans lesdites bactéries ou cellules eucaryotes, et dans lequel est insérée une séquence nucléotidique telle que définie ci-dessus, lequel vecteur est un vecteur d'expression d'un peptide, d'un fragment de peptide ou d'une combinaison de fragments de peptides ayant une activité de récepteur β3-adrénergique murin.

Selon une disposition avantageuse de ce mode de réalisation, ledit vecteur d'expression est constitué successivement d'une origine de réplication approprié, d'un gène de résistance à l'ampicilline, du promoteur viral SV40, d'une séquence nucléotidique conforme à l'invention codant pour un peptide ayant une activité de récepteur β3-adrénergique murin, d'un site de polyadénylation dérivé du virus de l'hépatite B (HBsAg) et du gène codant pour la dihydrofolate réductase (DHFR), enzyme qui permet la survie des cellules dans un milieu dépourvu en glycine, hypoxanthine et thymidine.

Un tel plasmide a été dénommé pSTH-Moβ3 par les Inventeurs et a été déposé sous le n° I-1026 en date du 14 janvier 1991 auprès de la Collection Nationale de Cultures de Microorganismes tenue par l'INSTITUT PASTEUR.

La présente invention a également pour objet une cellule hôte appropriée, obtenue par transformation génétique, caractérisée en ce qu'elle est transformée par un vecteur d'expression conforme à l'invention.

Une telle cellule est capable d'exprimer un peptide, d'origine murine, ayant une activité de récepteur β3-adrénergique.

Selon un mode de réalisation avantageux, la cellule hôte est notamment constituée par les cellules de la lignée CHO (Chinese Hamster Ovary).

Un autre des microorganismes utilisés peut être constitué par une bactérie, notamment *Escherichia coli*.

La présente invention a également pour objet un processus d'expression d'un peptide conforme à l'invention, caractérisé en ce que la cellule hôte, résultant de la transformation par un vecteur contenant une séquence nucléotidique, codant pour un peptide d'origine murine ayant une activité de récepteur β3-adrénergique, est cultivée de manière à produire et à transporter ledit peptide exprimé vers la membrane, de telle sorte que les séquences transmembranaires dudit récepteur β3 soient exposées à la surface de la membrane de l'hôte cellulaire transformé.

Dans le cas de l'expression en cellules eucaryotes, les éléments de régulation peuvent comporter le promoteur endogène des récepteurs adrénergiques ou des promoteurs viraux tels que ceux des virus SV40 ou du virus du sarcome de Roux (RSV).

Dans le cas de l'expression dans *E. coli*, les éléments de régulation peuvent comporter le promoteur de l'opéron lactose ou de l'opéron tryptophane.

La présente invention a également pour objet un modèle d'étude des récepteurs β3-adrénergiques, caractérisé en ce qu'il est constitué par des cellules hôtes conformes à l'invention, c'est-à-dire exprimant un récepteur β3-adrénergique murin à la surface de leur membrane cellulaire.

Un tel modèle permet l'étude, d'un point de vue pharmacologique, des récepteurs β3-adrénergiques murins, notamment en permettant l'identification des ligands β adrénergiques plus affins pour le récepteur β3 adrénergique ou plus sélectifs par rapport aux récepteurs β1 et β2 et ainsi la mise au point des médicaments actifs dans des maladies telles que le diabète et/ou l'obésité.

L'invention a également pour objet un procédé de détection de la capacité d'une substance à se comporter comme ligand vis-à-vis d'un peptide conforme à l'invention, lequel procédé comprend :
- la mise en contact de ladite substance avec une cellule hôte préalablement transformée par un vecteur d'expression conforme à l'invention, laquelle cellule hôte exprime ledit peptide murin (RAβ3 murin), le cas échéant après induction physique ou chimique appropriée, et laquelle mise en contact est réalisée dans des conditions permettant la formation d'une liaison entre l'un au moins des sites spécifiques et ladite substance s'il y a lieu ; et
- la détection de la formation éventuelle d'un complexe du type ligand-peptide.

La présente invention a, de plus, pour objet une méthode de production de souris recombinantes, n'exprimant plus le récepteur β3-adrénergique, caractérisée en ce que :
- l'on réalise, dans la séquence de formule I, conforme à l'invention, une mutation convenable qui inhibe l'expression du gène codant pour le récepteur β3-adrénergique murin ;
- l'on introduit ladite séquence I modifiée dans un segment de l'ADN génomique de souris, associé à un marqueur approprié, de manière à obtenir une séquence marquée M, contenant la séquence de formule I modifiée ;
- l'on intègre ladite séquence M, *in vitro,* dans les cellules souches de lignées germinales d'embryon de souris ; puis
- l'on réinjecte lesdites cellules souches à une souris ; et après recombinaison homologue,
- l'on obtient à la génération F2 des souris recombinées homozygotes, reconnaissables à la présence du marqueur.

Un protocole de souris recombinantes est, par exemple, décrit dans Nature, 1988, 336, 348-352.

Selon un mode de mise en oeuvre avantageux de ladite méthode, ledit segment d'ADN génomique de souris comprend au plus 14 Kbases.

Selon une disposition avantageuse de ce mode de mise en oeuvre, lorsque ledit fragment est de 14 Kbases, il comprend notamment les sites de restriction suivants : 3 sites SphI, 2 sites BamHI, 2 sites HindIII, 4 sites XbaI, 4 sites SacI, 1 site KpnI, 1 site SmaI, 2 sites BglII, 2 sites PvuII, 1 site PstI et 1 site EcoRI.

Selon un autre mode de mise en oeuvre avantageux de ladite méthode, la séquence de formule I mutée est comprise entre un site BglII et un site BamHI du segment d'ADN génomique de souris.

Les souris transgéniques et les souris recombinantes telles que définies ci-dessus, sont un modèle d'étude particulièrement avantageux, d'un point de vue physiologique, des récepteurs β3-adrénergiques.

La présente invention a, en outre, pour objet un procédé pour l'étude de l'affinité d'un polypeptide conforme à l'invention pour un ou plusieurs ligands déterminés, lequel procédé comprend :
- la transformation d'une cellule hôte appropriée par un vecteur d'expression conforme à l'invention ;
- la culture de la cellule hôte transformée, dans des conditions permettant l'expression du récepteur β3 codée par la séquence nucléotidique, et le transfert du récepteur β3 exprimé vers la membrane de ladite cellule, de sorte que les séquences transmembranaires du récepteur β3 soient exposées à la surface de la cellule hôte transformée ;
- la mise en contact de ladite cellule avec les ligands déterminés ; et
- la détection d'une réaction affine entre ladite cellule transformée et lesdits ligands déterminés.

L'invention a, en outre, pour objet un kit pour la détection de l'affinité éventuelle d'un ligand pour un peptide conforme à l'invention, lequel kit comprend :
- une culture de cellules hôtes transformées par un vecteur d'expression conforme à l'invention ;
- éventuellement, si nécessaire, des moyens physiques ou chimiques pour induire l'expression d'un peptide (récepteur β3 murin ou un fragment de celui-ci) codé par une séquence nucléotidique conforme à l'invention, contenue dans un vecteur dont le promoteur est inductible ;
- un ou plusieurs ligands témoins ayant des affinités déterminées pour ledit peptide ; et
- des moyens physiques ou chimiques pour la caractérisation de l'activité biologique du peptide exprimé.

L'invention a également pour objet des anticorps dirigés de façon spécifique contre l'un des peptides conformes à l'invention, ces anticorps étant tels qu'ils ne reconnaissent ni le récepteur adrénergique β1, ni le récepteur adrénergique β2.

Selon un mode de réalisation avantageux, lesdits anticorps sont des anticorps polyclonaux.

Selon un mode de réalisation avantageux, lesdits anticorps sont des anticorps monoclonaux.

On prépare les anticorps monoclonaux par fusion cellulaire entre des cellules de myélome et des cellules spléniques de souris immunisées, selon les procédés classiques.

De manière inattendue, le récepteur β3-adrénergique murin permet la réalisation de toutes les applications précitées.

Outre les dispositions qui précèdent, l'invention comprend encore d'autres dispositions, qui ressortiront de la description qui va suivre, qui se réfère à des exemples de mise en oeuvre du procédé objet de la présente invention, avec référence aux dessins annexés, dans lesquels :
- la figure 1 représente la carte de restriction du clone MB1 qui contient le gène β3-adrénergique murin et comprend 14 Kb ;
- la figure 2 représente les sites uniques de restriction de la séquence de formule I (gène murin β3) ;
- la figure 3 est une comparaison des régions codantes des gènes β3 humain et murin ;
- la figure 4 est une comparaison des récepteurs β3 humain et murin ;
- la figure 5 est une comparaison des récepteurs β1, β2 et β3 humains avec le récepteur β3 murin ;
- la figure 6 représente la détection par autoradiographie de l'ADN génomique de souris, s'hybridant avec des sondes spécifiques du gène β3-adrénergique ;
- la figure 7 est une représentation du plasmide pSTH-Mµβ3 ;
- la figure 8 illustre la localisation du gène β3-adrénergique murin sur le chromosome 8 par hybridation *in situ* ;
- la figure 9 illustre la liaison à saturation de l'[¹²⁵I] ICYP aux cellules intactes CHO-Muβ3 ;
- les figures 10 (10A, 10B et 10C) illustrent l'accumulation d'AMPc après exposition des cellules CHO-Muβ3 aux agonistes β classiques (A), aux agonistes β atypiques (B) et aux antagonistes β (C), en présence de (-)isoprotérénol 5 nM ;
- la figure 11 représente l'hybridation en Northern Blot de la sonde A21 avec l'ARN total de différents tissus de souris.

Il doit être bien entendu, toutefois, que ces exemples sont donnés uniquement à titre d'illustration de l'objet de l'invention, dont ils ne constituent en aucune manière une limitation.

### Exemple 1 : Isolement et identification du gène β3-adrénergique murin.

Le gène β3 murin a été isolé d'une banque d'ADN génomique de souris de la lignée NIH-3T3 construite dans le phage λ-FixII, en utilisant comme sonde un fragment d'ADN de 1034 pb (sonde Huβ3) contenant la totalité du gène β3-adrénergique humain précédemment isolé (EMORINE et al., Science, 245, 1118-1121, 1989). Après hybridation avec la sonde radiomarquée, les filtres sont lavés, et exposés une nuit sur film d'autoradiographie. 24 signaux d'hybridation ont été observés, dont 18 se sont par la suite révélés être de faux positifs. Les 6 clones restants (MB1, MB2, MB3, MB4, MB9, MB12) ont été purifiés par 4 sous-clonages successifs suivis d'une hybridation avec la sonde Huβ3 (voir Demande française 89 00918).

Pour identifier le(s) clone(s) contenant le gène β3-adrénergique murin, deux méthodes ont été utilisées : l'hybridation avec d'autres sondes β-adrénergiques, et l'amplification par PCR. L'ADN des 6 clones a été préparé, coupé par des endonucléases de restriction, puis hybridé avec plusieurs sondes dérivées des gènes β-adrénergiques, comme visible sur le Tableau I ci-après.

**TABLEAU I**

| SONDES | | | | | | |
|---|---|---|---|---|---|---|
| | Huβ3 | Huβ1 | Huβ2 | i3β3 | 3'β1 | i3β2 |
| Gène | | | | | | |
| | | | | | | |
| β1 humain | ++ | +++ | ++ | - | + | - |
| β2 humain | + | ++ | +++ | - | - | + |
| β3 humain | +++ | ++ | + | + | - | - |

| Clone | | | | | | |
|---|---|---|---|---|---|---|
| | | | | | | |
| MB1 | +++ | + | - | + | - | - |
| MB2 | +++ | + | - | + | - | - |
| MB3 | +++ | + | - | + | - | - |
| MB4 | +++ | + | - | + | - | - |
| MB9 | +++ | + | - | + | - | - |
| MB12 | + | - | - | - | - | - |

Les sondes utilisées dérivent toutes des gènes β-adrénergiques humains, β1, β2 ou β3, telles que décrites dans la Demande de Brevet français n° 89 00918.

Les sondes Huβ1, Huβ2 et Huβ3 (environ 1,2 Kb) contiennent respectivement la totalité des régions codantes des gènes β1, β2 et β3. Comme l'indique la partie supérieure du Tableau I, chacune des sondes hybride avec les trois gènes humains.

Les sondes 3'β1, i3β2 et i3β3 (environ 200 à 300 pb) correspondent respectivement à la partie carboxyterminale du β1AR, et à la troisième boucle intracellulaire des β2AR et β3AR. Ces sondes dérivent de régions très divergentes entre les gènes β1, β2 et β3, et sont donc spécifiques : comme l'indique la partie supérieure du Tableau I, chacune des sondes n'hybride qu'avec le gène correspondant.

Il ressort de ce qui précède, que les clones MB1, MB2, MB3, MB4 et MB9 hybrident très fortement avec la sonde Huβ3, et hybrident également avec la sonde i3β3. Le clone MB12 hybride très faiblement avec la sonde Huβ3 de départ, et pas du tout avec la sonde spécifique i3β3. D'autre part, la comparaison des profils de digestion de ces six clones avec plusieurs enzymes de restriction montre que MB1, MB4, MB9 d'une part, et MB2, MB3 d'autre part, sont identiques, et que ces deux groupes de clones sont chevauchants. Les clones MB1, MB4 et MB9 sont les plus intéressants car la région hybridant avec la sonde est située à peu près au milieu du fragment d'ADN cloné.

Pour montrer que MB1 contient bien le gène β3-adrénergique murin, des expériences d'amplification par PCR ont été réalisées en utilisant comme amorces, soit des oligonucléotides "dégénérés", situés dans les domaines les plus conservés entre les différents gènes β-adrénergiques, soit des oligonucléotides "spécifiques" du gène β3-adrénergique humain, dérivés des séquences les plus divergentes entre les trois gènes. Dans les deux cas, un produit 'd'amplification de la taille attendue a pu être obtenu en utilisant comme matrice l'ADN du clone MB1.

De ces deux expériences, hybridation et amplification, il a pu être déduit que les clones de la famille MB1 contiennent le gène β3-adrénergique murin.

### Exemple 2 : Localisation du gène β3-adrénergique dans le clone MB1.

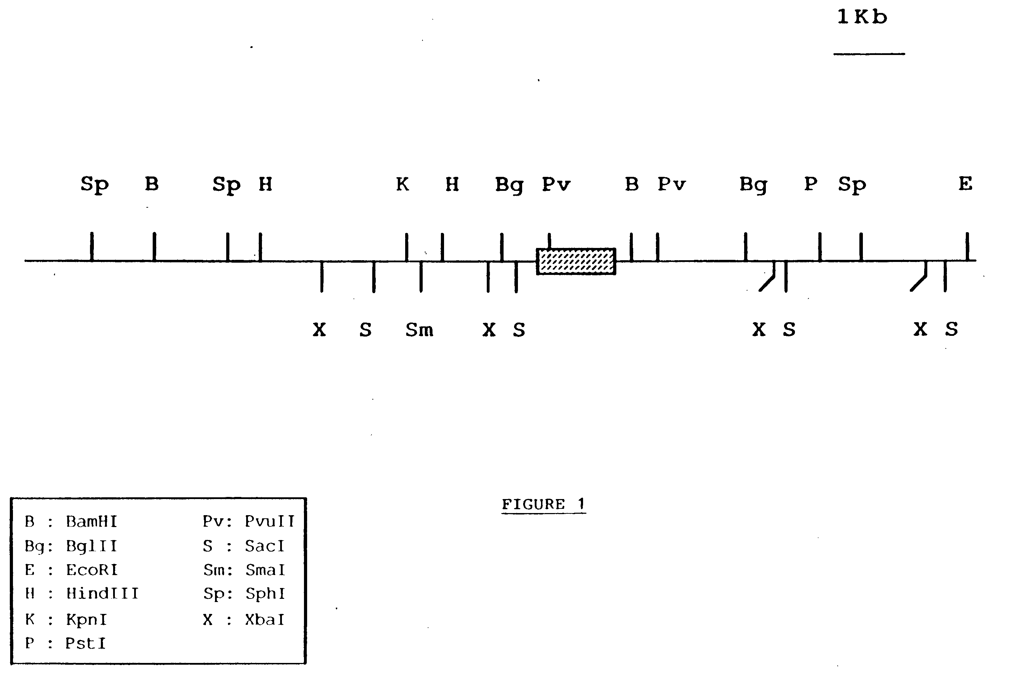

L'ADN du clone MB1, qui comprend 14 Kb, a été digéré par douze enzymes de restriction, soit indépendamment, soit deux à deux. Les fragments résultant ont été déposés sur gel d'agarose, transférés sur membrane de nylon, et hybridés avec la sonde Huβ3. Après lavage et exposition sur film d'autoradiographie, les fragments hybridant avec la sonde ont pu être identifiés, et la carte de restriction du clone MB1 a été établie (figure 1).

Dans le cas de l'utilisation d'une seule enzyme de restriction, on obtient un fragment de 3,6 kb avec BglII, de 13 kb avec PstI, de 4,1 kb avec SacI et de 6,9 kb avec BamHI.

Le plus petit fragment d'ADN hybridant avec la sonde Huβ3 est un fragment d'environ 1,7 Kb délimité par les sites SacI et BamHI. C'est donc dans ce fragment qu'est localisé le gène β3-adrénergique murin.

### Exemple 3 : Séquençage du gène β3-adrénergique murin.

On a séquencé le fragment d'ADN d'environ 2 Kb délimité par les sites des enzymes BglII et BamHI (voir figure 1).

Ce fragment d'ADN a été purifié à partir de l'ADN du clone MB1, et sous-cloné dans le site BamHI du vecteur M13mp18. Les clones M13 ayant intégré le fragment d'ADN dans les deux orientations opposées ont été identifiés (clones MB1BB1 et MB1BB2). L'ADN de ces deux clones M13 a été préparé et délété avec l'exonucléase III (ExoIII). Plusieurs délétants ont été isolés, leur ADN simple brin a été préparé et séquencé par la méthode de Sanger.

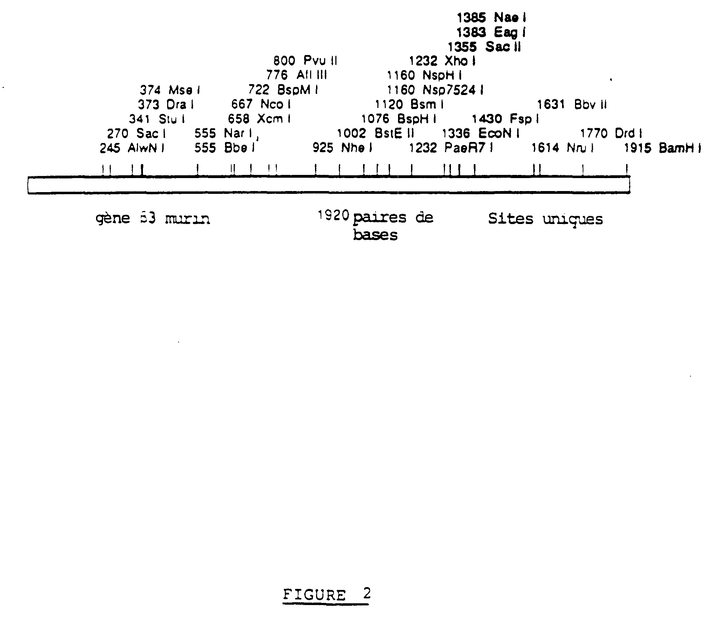

La séquence nucléotidique âu gène β3 de souris (1180 bp) et des régions non codantes (555 bp en 5' et 186 bp en 3' du gène) est représentée par la formule I. Les sites de restriction contenus dans ce fragment de 1920 bp sont positionnés sur la figure 2.

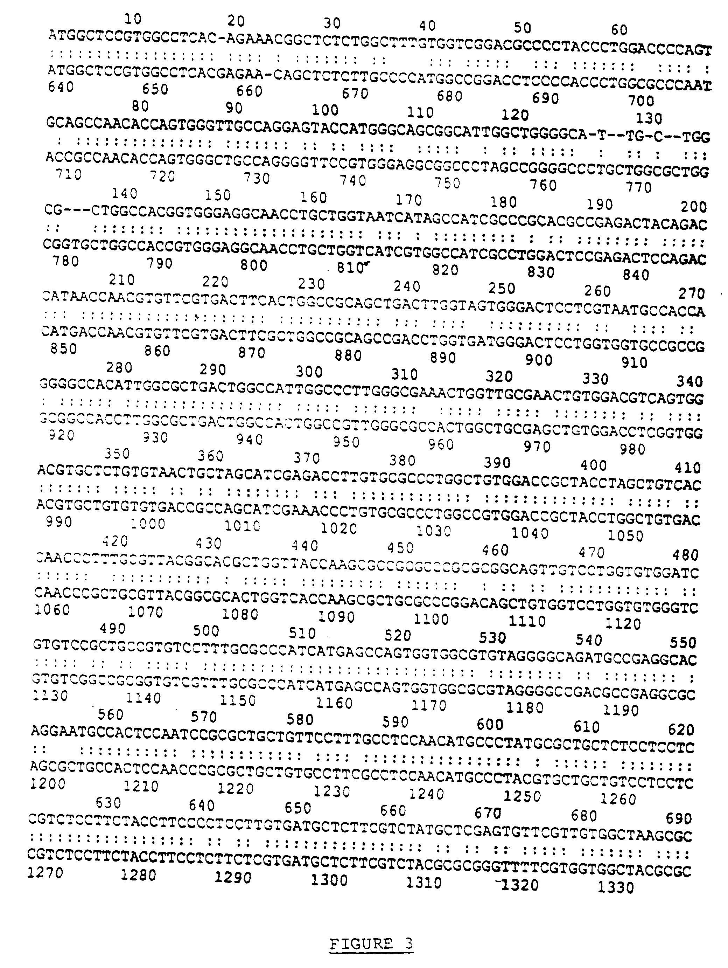

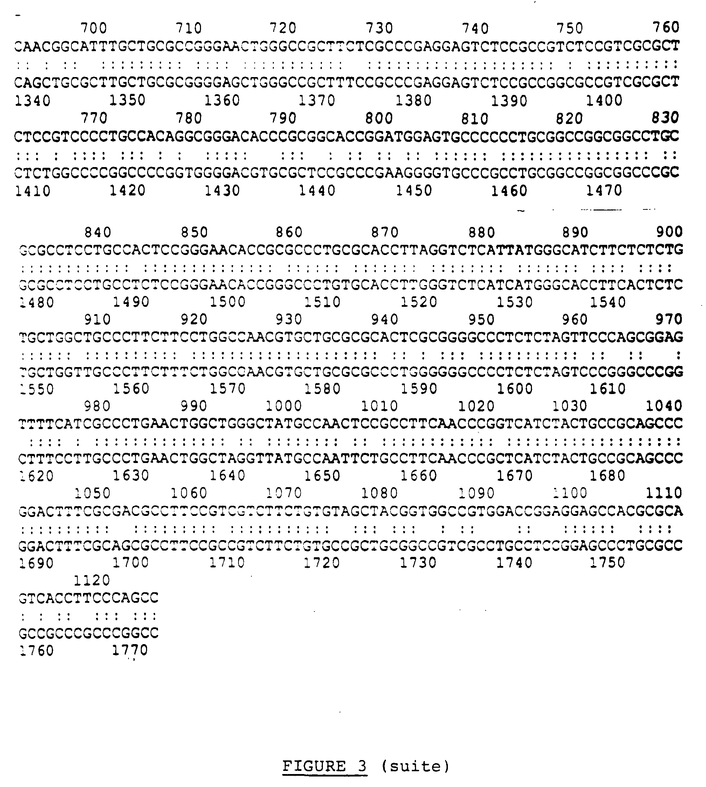

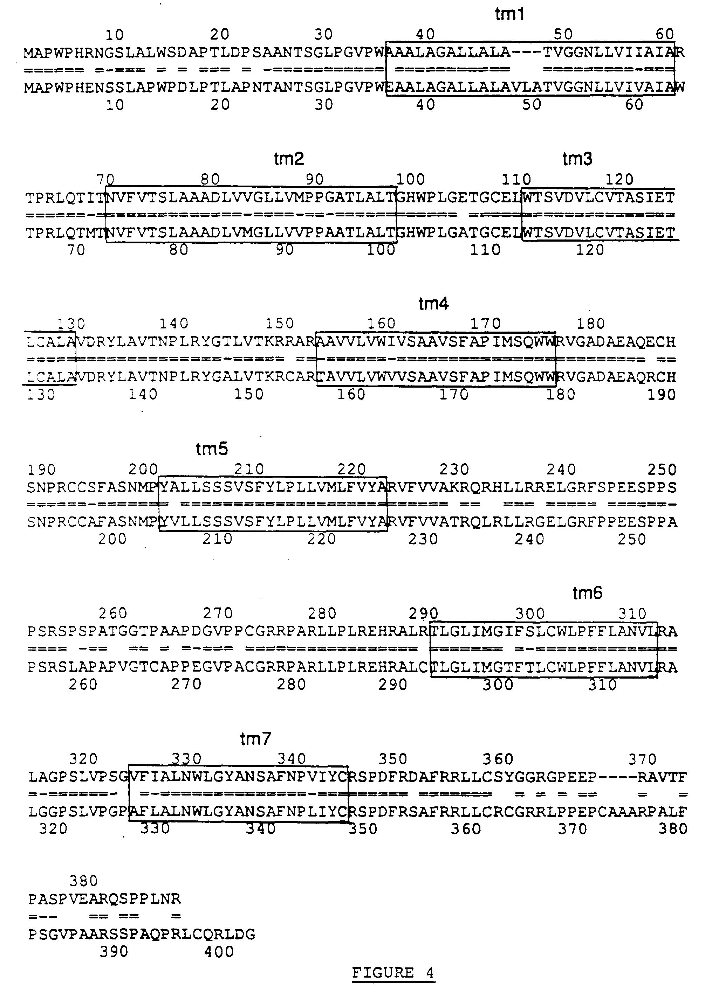

La comparaison des régions codantes des gènes β3 humain et murin indique une conservation de 82 % en nucléotides (figure 3) alors qu'ils ne présentent qu'une homologie de l'ordre de 48-55 % avec les gènes RA-β1 et β2 de différentes espèces. Le gène β3 murin ne contient pas d'intron, et code pour un polypeptide de 388 acides aminés qui présente 82 % d'homologie avec le polypeptide β3 humain (figure 4). Cette homologie est principalement concentrée dans les régions transmembranaires (93 %) ("TM").

### Exemple 4 : Préparation de sondes spécifiques du gène β3-adrénergique murin.

Trois fragments d'ADN ont été purifiés dans le but d'obtenir une sonde spécifique du gène β3-murin.

La sonde "A19" correspond au fragment BglII-BamHI de 2 Kb qui a été séquencé. Ce fragment a été purifié par migration sur gel d'agarose, électroélution et passage sur une colonne échangeuse d'ions (NACS).

La sonde "A20" correspond au produit d'amplification obtenu par PCR à partir du gène β3-murin en utilisant comme amorces les oligonucléotides "dégénérés" (5'-AAGCTTGT/CGTG/CACG/CGCCAGCATC/TGAG/AACCCTGTG-3' et 5'-GTCGACAAGAAGGGCAGC/ACAGCAGAGG/C/AGTGAA-3' dans un milieu réactionnel comprenant : 100 mM Tris-HCl pH 8,4, 30 mM MgCl₂, 0,5 % Tween 20, 0,25 % Nonidet P40, 5 % Formamide et 10 % diméthylsulfoxide. Le fragment d'ADN obtenu (environ 600 pb) comprend la partie du gène contenue entre les régions transmembranaires TM3 et TM6. Après amplification, les extrémités du fragment A20 ont été remplies avec le fragment "Klenow" de la polymérase I. La sonde a alors été purifiée par migration sur gel de polyacrylamide, électroélution, et passage sur une colonne échangeuse d'ions.

La sonde "A21" correspond au produit d'amplification obtenu par PCR à partir du gène β3 murin en utilisant comme amorces des oligonucléotides "spécifiques" du gène β3 humain (5'-GCATGCTCCGTGGCCTCACGAGAA-3' et 5'-CTGCAGGAGGAGGACAGCAGCA-3') dans un milieu réactionnel comprenant 670 mM Tris-HCl pH 8,4, 67 mM MgCl₂, 1 mg/ml gélatine, 67 µM EDTA, 100 mM β-mercaptoéthanol, 160 mM (NH₄)₂SO₄, 5 % Formamide et 10 % diméthylsulfoxide. Le fragment d'ADN obtenu (environ 300 pb) comprend la région amino-terminale du gène, allant du codon d'initiation de la traduction jusqu'à la fin du domaine TM2. La sonde A21 a été purifiée de la même façon que la sonde A20.

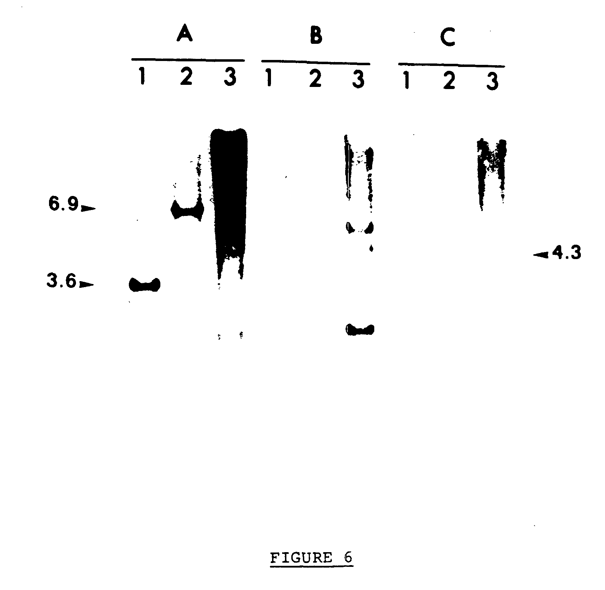

Les trois sondes ainsi préparées ont été radiomarquées et utilisées dans des expériences d'hybridation d'ADN génomique (Southern blot) pour estimer leur spécificité vis-à-vis du gène β3. Des ADN génomiques préparés à partir de foie de souris BALB/c, ont été digérés par une enzyme de restriction (BglII : traces 1 de la figure 6; BamHI : traces 2 de la figure 6) ; de même l'ADN génomique isolé à partir de cellules A431 (lignée épidermoide humaine), a été digéré par BamHI (traces 3 de la figure 6) ; les fragments obtenus ont été séparés sur gel d'agarose puis transférés sur membrane de nylon. La membrane a ensuite été hybridée avec une des sondes radiomarquées (A19 : zone A, A20 : zone B ou A21 : zone C de la figure 6), lavée à faible stringence puis autoradiographiée. Chacune de ces sondes détecte une bande unique sur l'ADN génomique de souris BALB/c (figure 6). Dans chacun des cas, la taille du fragment hybridé (3,6 Kb avec l'enzyme BglII, 6,9 Kb avec l'enzyme BamHI, 13 kb avec l'enzyme PstI et 4,1 kb avec SacI) correspond à celle du gène β3-adrénergique murin qui a été cloné.

Ces résultats indiquent que chacune de ces trois sondes détecte spécifiquement le gène β3-adrénergique de souris. La présence d'une bande unique dans l'ADN total de souris indique qu'il n'y a pas, dans le génome murin, d'autre séquence hautement homologue au gène β3-adrénergique. On peut en conclure qu'il existe chez la souris une seule copie du gène RA-β3.

### Exemple 5 : Construction d'un vecteur pour l'expression du RA-β3 murin.

L'étude des propriétés pharmacologique du produit du gène β3 murin exige qu'il soit exprimé à la surface de cellules eucaryotes, qui possèdent tous les éléments nécessaires à la transduction du signal. Pour pouvoir directement comparer les propriétés des récepteurs β3-AR murin et humain, on a choisi d'exprimer le gène β3 de souris dans les cellules de la lignée CHO (Chinese Hamster Ovary), déjà utilisées pour analyser le récepteur β3-AR humain (voir Demande de Brevet français n° 89 00918).

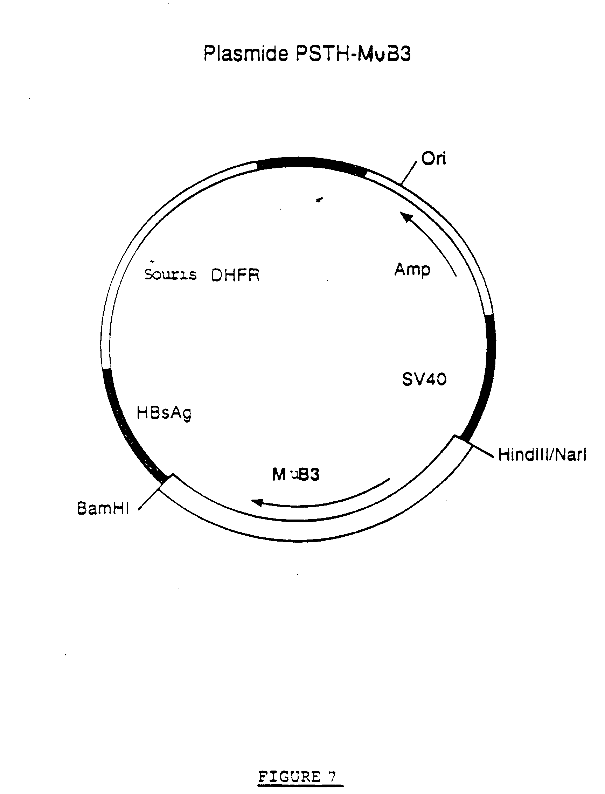

Dans ce système (figure 7), la région codante du gène à analyser est insérée entre le promoteur viral SV40 (ce promoteur fort permet d'obtenir un haut niveau d'expression) et un site de polyadénylation dérivé du virus de l'hépatite B (HBsAg), dans un plasmide comprenant également le gène codant pour la dihydrofolate réductase (DHFR, enzyme qui permet la survie des cellules dans un milieu dépourvu de glycine, d'hypoxanthine et de thymidine).

La carte de restriction du fragment de 2 Kb qui a été séquencé (figure 2) indique la présence d'un site de coupure par l'enzyme NarI à la position 555, soit 15 nucléotides en amont de la région codant du gène β3 de souris. L'ADN du clone MB1BB1 (2 Kb insérés dans M13mp18) a été digéré avec les enzymes NarI et BamHI, pour libérer le fragment de 1360 pb contenant la région codante du gène β3 et une partie de la région 3' non traduite. Ce fragment d'ADN a été purifié, puis inséré dans le vecteur d'expression aux sites de coupure des enzymes HindIII et BamHI (figure 7). Comme les extrémités générées par les enzymes NarI et HindIII ne sont pas compatibles, on a pris soin, au cours de la digestion enzymatique, de traiter les extrémités NarI de l'insert d'une part, et les extrémités HindIII du vecteur d'autre part, avec le fragment "Klenow" de la polymérase I, de façon à obtenir des bouts francs.

On a ainsi obtenu le plasmide recombinant pSTH-Mυβ3 représenté sur la figure 7.

### Exemple 6 : Localisation chromosomique du gène RA-β3 murin.

### - Protocole :

Pour la localisation chromosomique du gène RA-β3 murin, l'hybridation *in situ* est réalisée sur des lymphocytes, en métaphase, stimulés par la concanavaline A ; ces lymphocytes sont issus de souris mâles WMP, dans lesquelles tous les autosomes, à l'exception de l'autosom 19, sont sous la forme de translocations Robertsonienne.

Les lymphocytes sont cultivés à 37°C pendant 72 h, avec de la 5-bromodésoxyuridine (60 µg/ml) ajoutée à la 65ème heure de culture.

La sonde spécifique est la sonde A21 telle que décrite à l'exemple 4.

Cette sonde est sous-clonée dans le vecteur plasmidique pUC19, marquée au tritium par translation de coupure, de manière à obtenir une activité spécifique de 10⁸ d.p.m./µg et est utilisée à une concentration finale de 25 ng/ml de solution d'hybridation.

L'hybridation des cellules en métaphase, les lavages et la coloration sont réalisés comme décrit dans MATTEL et al. (Human Genetics, 1985, 69, 268-271).

### - Localisation :

Le gène RA-β3 murin a été localisé dans la région (8A2→8A4) du chromosome 8 par hybridation chromosomique *in situ,* conformément au protocole ci-dessus.

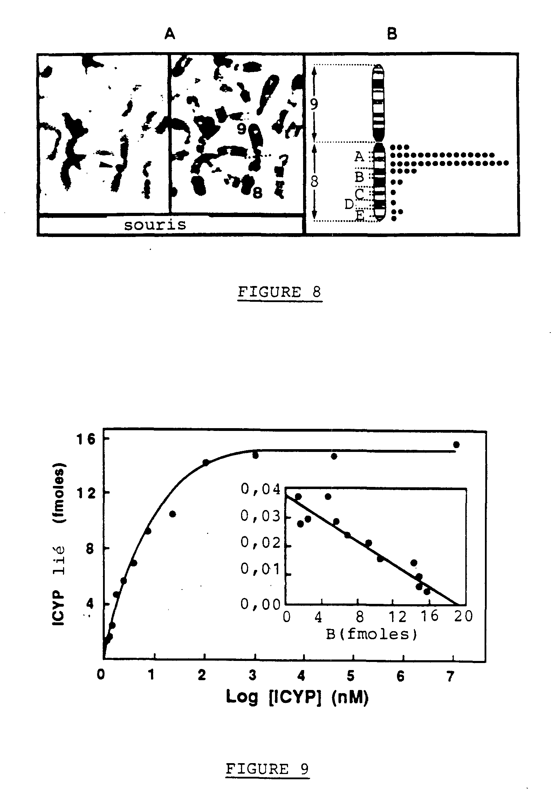

Dans les 150 cellules en métaphase examinées, il y avait 278 points colorés à l'argent, associés avec les chromosomes : 46 de ces points (16,5 %) étaient localisés sur les chromosomes 8, et 32 d'entre eux (69,5 %) dans la région (8A2→8A4) (figure 8).

La figure 8 comprend en 8A, une métaphase partielle de lymphocyte de souris, montrant le site spécifique de l'hybridation au chromosome 8 ; sur la gauche, la flèche indique les points colorés à l'argent sur les chromosomes colorés au Giemsa, après autoradiographie ; sur la droite (figure 8B), les chromosomes colorés sont identifiés par "R-banding".

### Exemple 7 : Pharmacologie du récepteur RA-β3 murin.

### 1. Tests mis en oeuvre :

a. test d'accumulation de l'AMPc :
   des cellules préconfluentes telles que celles décrites à l'exemple 5 (5.10⁵) sont incubées à 37°C pendant 20 minutes dans 0,5 ml de tampon de HANKS contenant de l'HEPES 20 mM (pH 7,4), de l'acide ascorbique 1 mM, de l'isobutylméthylxanthine 1 mM et différentes concentrations d'agoniste.
   Après ébullition pendant 5 min et centrifugation (4 000 r.p.m. 10 min, 4°C), la quantité d'AMPc produite est déterminée en utilisant le kit Amersham AMPc.
   Pour l'étude de l'inhibition de l'accumulation d'AMPc, les cellules sont préincubées à 37°C pendant 10 min avec un antagoniste, avant l'addition de (-)isoprotérénol 5 nM, suivie d'une incubation pendant 20 min.
b. test de liaison de l'ICYP :
   des cellules préconfluentes telles que celles décrites à l'exemp 5 (10⁵) sont incubées pendant 1 heure à 37°C dans un tampon HANKS contenant de l'HEPES 20 mM (pH 7,4), de l'acide ascorbique 1 mM, de la désipramine 2 µM, de la sérumalbumine bovine 0,05 % et du (-) [¹²⁵I]ICYP 70-7 000 pM (2 000 CI/mmol, Amersham).
   La liaison non spécifique est déterminée en présence de (-)isoprotérénol 100 µM.
   Les incubations sont arrêtées par dilution dans un tampon PBS froid et le ligand lié est séparé du [¹²⁵I]ICYP libre par filtration (filtres Whatman GF/C imbibés de polyéthylèneimine à 0,3 %).
   Les filtres sont lavés 3 fois avec du tampon PBS froid et la radioactivité est mesurée.

### 2. Profil pharmacologique :

Pour déterminer le profil pharmacologique du RA-β3 murin, la région codante de son gène a été insérée dans un vecteur d'expression tel que celui décrit à l'exemple 5.

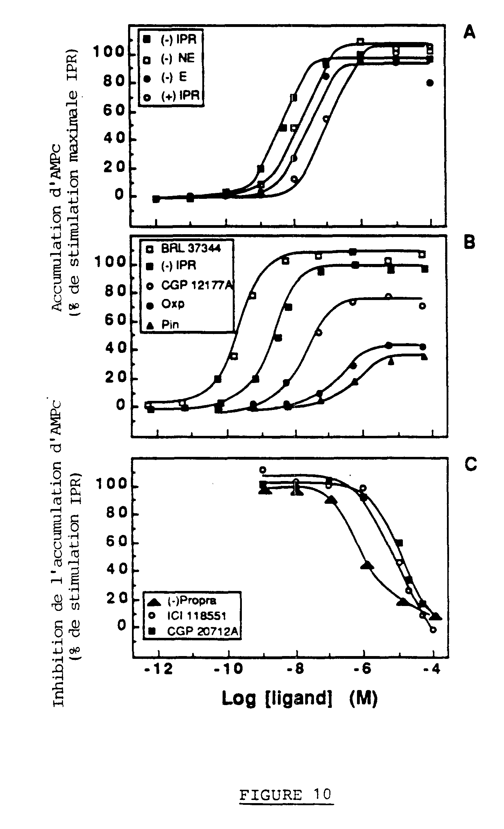

La liaison à saturation de [¹²⁵I]iodocyanopindolol (ICYP) et l'analyse Scatchard correspondante (figure 9, qui comprend en abscisse les log de concentration en ICYP (nM) et en ordonnées, la quantité d'ICYP lié (fmoles) (avec Scatchard)) indique que les cellules CHO transfectées de manière stable (CHO-Muβ3) expriment 200 000 ± 50 000 récepteurs/cellule avec une constante de dissociation de 880 ± 88 pM (moyenne ± SEM, n = 3).

L'accumulation d'AMPc est stimulée par l'incubation des cellules CHO-Muβ3 avec l'isoprotérénol, la noradrénaline et l'adrénaline (figure 10A).

Cette stimulation est stéréospécifique puisque le (-)isoprotérénol est 20 fois plus actif que le (+)isoprotérénol, comme l'illustre le Tableau II ci-après :

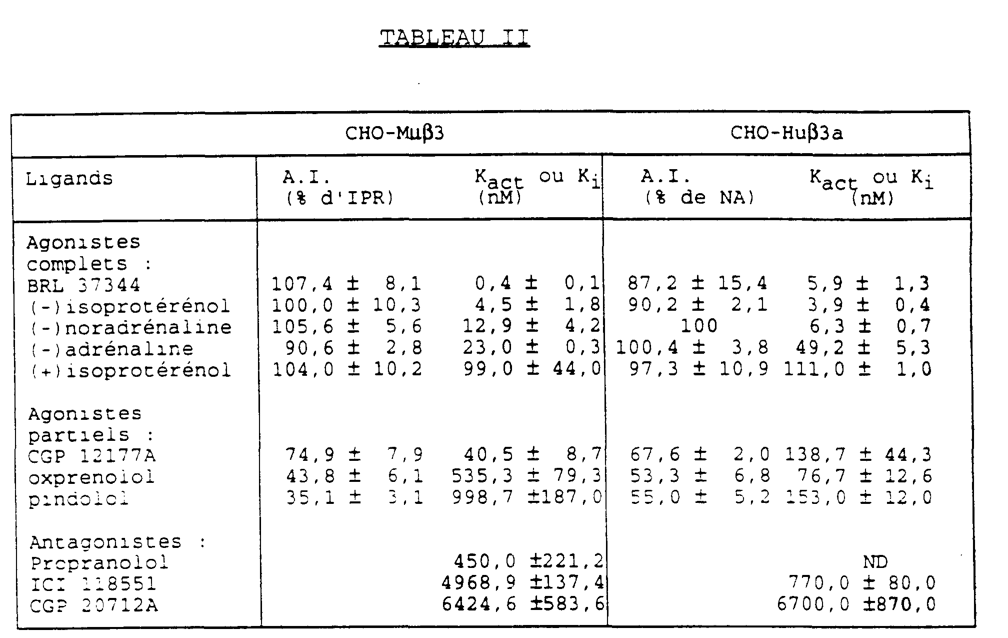

Sur ce Tableau II, A.I. représente l'activité intrinsèque et correspond au pourcentage de stimulation maximale obtenue pour chaque agoniste, en fonction de l'effet maximal du (-)isoprotérénol, les concentrations basale et maximale en AMPc, induit par l'isoprotérénol, étant respectivement de 25 et 500 pmol par million de cellules.

Les valeurs de K_{act} correspondent à la concentration en agoniste donnant 50 % de la stimulation maximale.

Les valeurs de Kᵢ sont calculées, conformément à CHENG et PRUSOFF (Biochem. Pharmacol., 1973, 22, 3099-3108), avec des C.I.₅₀ définies comme la concentration en antagoniste requise pour induire une inhibition de 50 % de l'activité maximale obtenue avec du (-)isoprotérénol (5 nM).

L'agoniste BRL 37344 est 10 fois plus actif que le (-)isoprotérénol (IPR), pour induire une accumulation d'AMPc (figure 10B, Tableau II).

Les antagonistes β1 et β2 CGP 12177A, oxprénolol et pindolol se comportent comme des agonistes partiels du RA-β3 murin, avec une stimulation maximale de l'accumulation en AMPc de 77 %, 45 % et 36 %, respectivement de celle du (-)isoprotérénol.

Pour d'autres antagonistes β tels que le propranolol (non-sélectif), l'ICI 118551 (β2-sélectif) et le CGP 20712A (β1-sélectif) (figure 10C), des concentrations micromolaires sont nécessaires pour inhiber l'accumulation d'AMPc induite par le (-)isoprotérénol.

Les figures 10A et 10B comprennent en abscisse le log de la concentration en ligand (M) et en ordonnée le pourcentage d'accumulation en AMPc (pourcentage de la réponse maximale induite par l'isoprotérénol).

La figure 10A correspond aux β-agonistes classiques (■ : (-)isoprotérénol ; □ : (-)noradrénaline ; ● : (-)adrénaline ; ○ : (+)isoprotérénol) ; la figure 10B correspond à des β-agonistes atypiques (□ : BRL 37344 ; **■** : (-)isoprotérénol ; ○ : CGP 12177A ; **●** : oxprénolol ; ▲ : pindolol).

La figure 10C comprend en abscisse le log de *la concentration* en ligand (M) *et en ordonnée le* pourcentage d'inhibition de l'accumulation d'AMPc (pourcentage de la réponse induite par l'isoprotérénol 5 nM) et correspond à l'action des antagonistes β en présence de (-)isoprotérénol 5 nM (▲ : (-)propranolol ; ○ : ICI 118551 ; ■ : CGP 20712A).

### 3. Distribution tissulaire :

L'analyse par hybridation avec la sonde A21, de l'ARN total (technique de Northern), extrait de différents tissus de souris, révèle une bande majeure à 2,2 kb aussi bien dans le tissu adipeux blanc que dans le tissu adipeux brun.

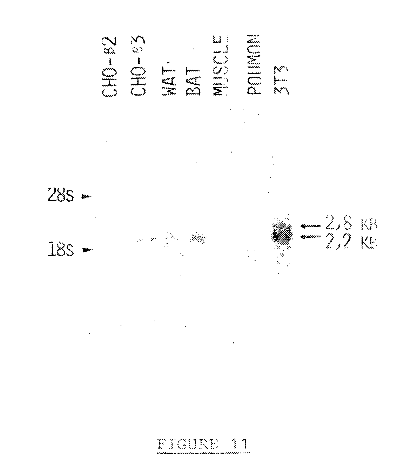

Dans les adipocytes différenciés 3T3-F442A, un transcrit majeur de taille similaire (2,2 kb) aussi bien qu'un transcrit mineur de 2,8 kb sont détectés (figure 11).

On ne détecte aucun signal d'hybridation dans l'ARN total préparé à partir d'autres tissus (muscle fémoral, poumon, foie, iléum, colon, cerveau, estomac, coeur et rein).

Le profil pharmacologique ci-dessus du RA-β3 murin évoque un récepteur agissant sur la lipolyse (récepteur du type RA-β atypique impliqué dans le contrôle des dépenses énergétiques des cellules adipeuses de rongeur).

Par exemple, l'effet agoniste puissant de BRL 37344 sur le RA-β3 rappelle son efficacité pour la stimulation de l'accumulation d'AMPc, de la lipolyse et de la thermogénèse chez les cellules de rat obèse.

L'agoniste spécifique du RA-β3 CGP 12177A stimule la lipolyse et l'activité adénylate cyclase.

La figure 11 représente une hybridation entre la sonde A21 et l'ARN total (30 µg) de tissus de souris ou de lignées cellulaires adipeuses 3T3-F442A (au jour 7 de la différentiation), après transfert d'ARN (technique de Northern).

Dans cette figure 11, WAT signifie tissu adipeux blanc ; BAT signifie tissu adipeux brun.

Les tailles des transcrits s'hybridant sont indiqués en kb, sur la droite et la position des bandes d'ARNr 28S et 18S est représentée sur le côté gauche de la figure.

### 4. Comparaison avec le RA-β3 humain :

### a. Points communs :

La comparaison des propriétés pharmacologiques est illustrée au Tableau II ci-dessus.

L'antagoniste β-adrénergique manqué [¹²⁵I]ICYP se lie au RA-β3 murin et au RA-β3 humain avec une affinité similaire, qui est de l'ordre de 10 à 20 fois inférieure à celle obtenue avec les RA-β1 et RA-β2.

De la même manière, les antagonistes tels que le propranolol, l'ICI 118551 et le CGP 20712A sont des inhibiteurs faibles de l'accumulation d'AMPc induite par l'isoprotérénol.

Comme le récepteur humain, le RA-β3 murin peut être activé par les antagonistes β1 et β2 : CGP 12177A, oxprénolol et pindolol.

Ces deux types de récepteurs (murin et humain) présentent une stéréosélectivité faible.

### b. Différences :

Dans les domaines transmembranaires, qui sont impliqués dans la liaison au ligand, on note la présence de 12 substitutions entre les deux récepteurs.

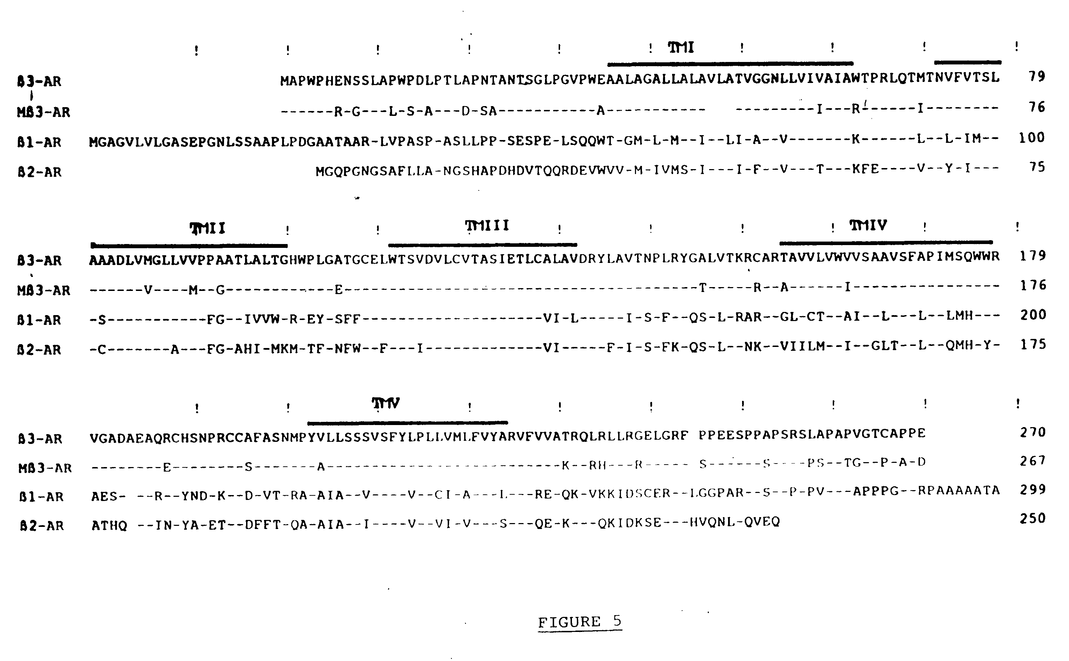

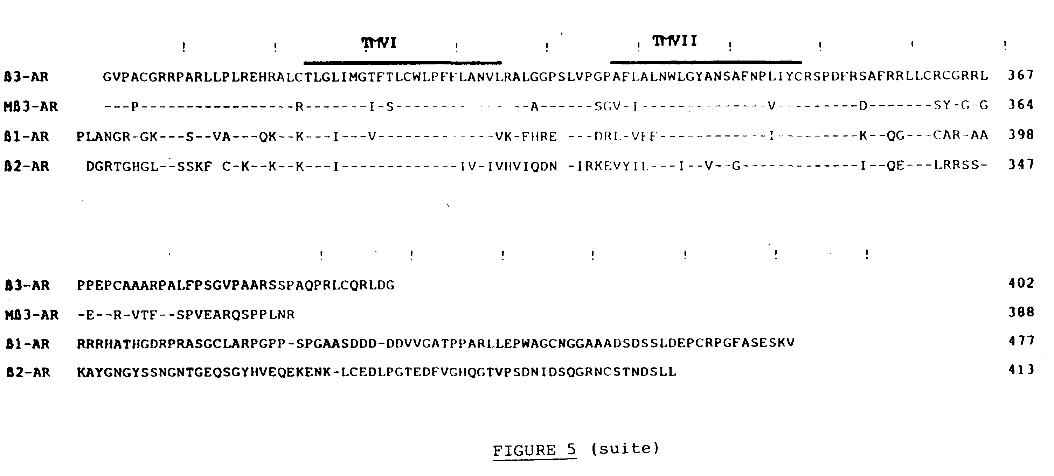

De plus, les 30 résidus C-terminaux du β3 de souris ne présentent pas d'homologie avec la queue C-terminale du récepteur β3 humain (figure 5). Le RA-β3 murin comprend un résidu tyrosine en position 360 de la séquence en acides aminés, qui n'existe pas dans la séquence du RA-β3 humain (figure 5).

Les différences les plus marquantes apparaissent dans les boucles intracytoplasmiques près des régions transmembranaires, où 3 résidus arginine (positions 61, 150 et 289 du RA-β3 murin) sont remplacés par un tryptophane, une cystéine et une autre cystéine, respectivement dans le RA-β3 humain.

De telles différences pourraient être à l'origine des modifications pharmacologiques telles que celle de la puissance accrue de l'agoniste BRL 37344 chez la souris, qui permet notamment, l'étude du métabolisme du tissu adipeux ; en effet, le RA-β3 murin est, de manière inattendue, suffisamment proche du RA-β3 humain pour une telle étude.

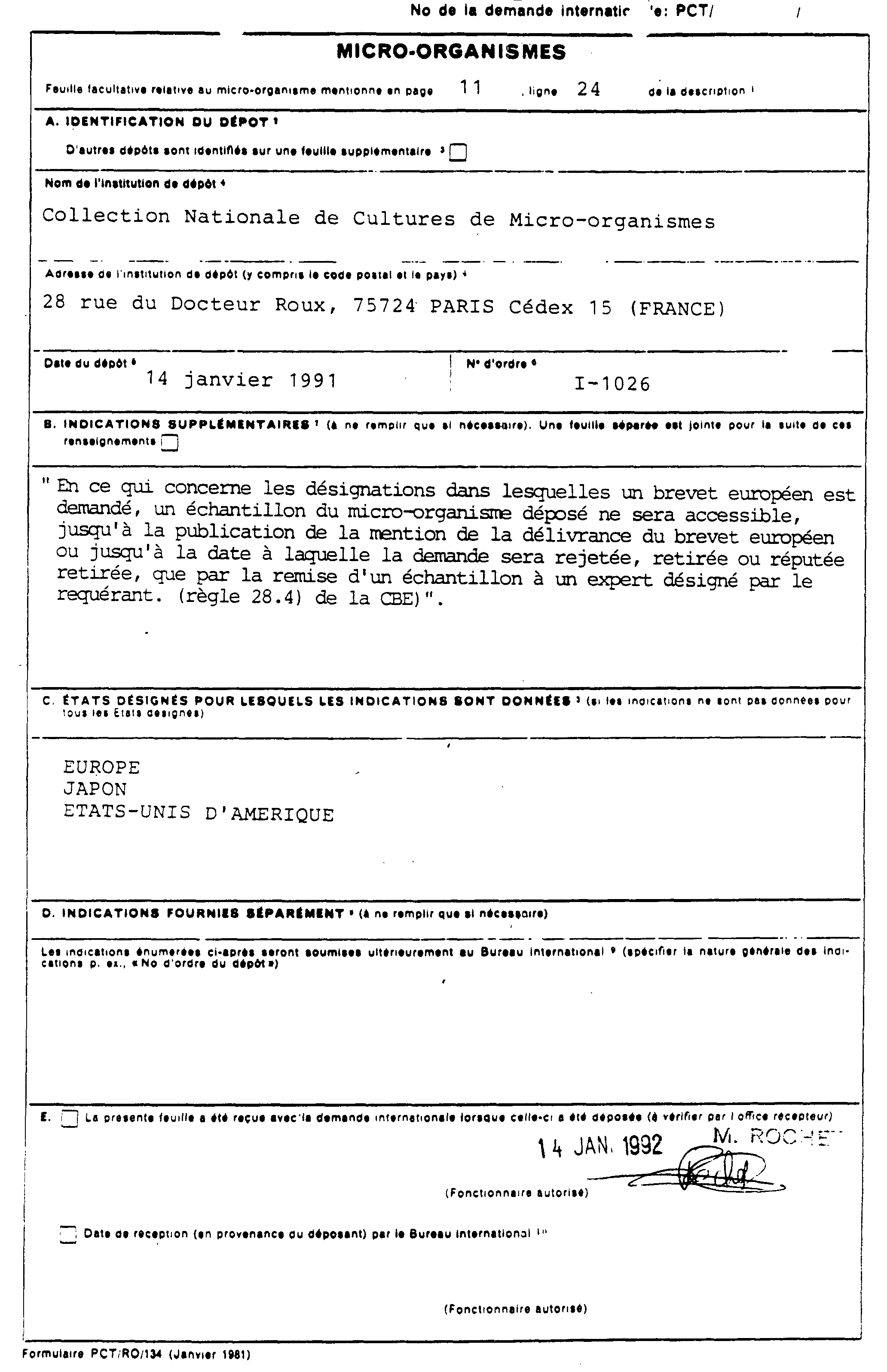
No de la demande internatic 'e: PCT/ /
MICRO-ORGANISMES
Feuille facultative relative au micro-organisme mentionne en page 11 , ligne 24 de la description ¹
A. IDENTIFICATION DU DÉPOT ¹
D'autres dépôts sont identifiés sur une feuille supplémentaire ³ [ ]
Nom de l'institution de dépôt ⁴
Collection Nationale de Cultures de Micro-organismes
Adresse de l'institution de dépôt (y compris le code postal et le pays) ⁴
28 rue du Docteur Roux, 75724 PARIS Cédex 15 (FRANCE)
Date du dépôt ⁵ | N° d'ordre ⁶
14 janvier 1991 | I-1026
B. INDICATIONS SUPPLÉMENTAIRES ⁷ (à ne remplir que si nécessaire). Une feuille séparée est jointe pour la suite de ces
renseignements [ ]
" En ce qui concerne les désignations dans lesquelles un brevet européen est
demandé, un échantillon du micro-organisme déposé ne sera accessible,
jusqu'à la publication de la mention de la délivrance du brevet européen
ou jusqu'à la date à laquelle la demande sera rejetée, retirée ou réputée
retirée, que par la remise d'un échantillon à un expert désigné par le
requérant. (règle 28.4) de la CBE)".
C. ÉTATS DÉSIGNÉS POUR LESQUELS LES INDICATIONS SONT DONNÉES ³ (si les indications ne sont pas données pour
tous les États designés)
EUROPE
JAPON
ETATS-UNIS D'AMERIQUE
D. INDICATIONS FOURNIES SÉPARÉMENT ⁸ (à ne remplir que si nécessaire)
Les indications énumerées ci-après seront soumises ultérieurement au Bureau International ⁹ (spécifier la nature générale des indi-
cations p. ex., «No d'ordre du dépôt»)
E. [ ] La presente feuille a été reçue avec la demande internationale lorsque celle-ci a été déposée (à vérifier par l office récepteur)
14 JAN. 1992 M. ROCHE[?][?]
(Fonctionnaire autorisé)
[ ] Date de reception (en provenance du déposant) par le Bureau International ¹⁰
(Fonctionnaire autorisé)
Formulaire PCT/RO/134 (Janvier 1981)

## Revendications

1. Séquence d'acide nucléique, **caractérisée en ce qu'**elle comprend le gène murin codant pour un peptide ayant une activité de récepteur β3-adrénergique, et **en ce qu'**elle comporte 1920 paires de bases et comprend la séquence en nucléotides de formule I ci-après :

GATCTGTAATCCCAGCACTGGGGAGGTTGAGGCAGAAGGATCTGGAGGTCCAGACCAATCTGGGCAACATATAGAAAGACTATCTCAAACAATAAGATAC 100

CTTAGGGAGAGCATCCAAGCAGAAGAGGGGCTATCTTGGATGGTTTGGCTTGTTCGGTTTTGTTTTGGTTTGTTTCTGGATGGTTGCCTTCCTTGTTCGG 200

TAAAGGATAGGGTGCGGGGGTTTCTCTTCTTTGCAGGGTTGCCTCAGCTTCTCCCAGCAAGCAGCTGCTGAGCTCCAGGAAACCGGTGCTCAGCGAGTCT 300

CAAGACAGGACGCCCCCTCTCCACCCTCCAATTCCACCAGAGGCCTCTCTTGTCACTATTGGACCCTGTTCCTTTAAAAGCAGCCACTCCTCCCGGCAAC 400

TAGGGTCTACATGGGGGGTGAGATGGAGGGAAGCTGACAGACTTACCCCACCAATTAGGGAAGATGGCCCAGGCTGGAAGAGTCGCTCCCAAGCCCTACT 500

GTCCCCTTCCCTAAGCCAGCGGGTCTGGGACGACGGGGAACCTTCCCACCCCAGGCGCCACACGAG ATG GCT CCG TGG CCT CAC AGA AAC 591

GGC TCT CTG GCT TTG TGG TCG GAC GCC CCT ACC CTG GAC CCC AGT GCA GCC AAC ACC AGT GGG TTG CCA GGA GTA 666

Tn1
CCA TGG GCA GCG GCA TTG GCT GGG GCA TTG CTG GCG CTG GCC ACG GTG GGA GGC AAC CTG CTG GTA ATC ATA GCC 741

Tn1 Tn2
ATC GCC CGC ACG CCG AGA CTA CAG ACC ATA ACC AAC GTG TTC GTG ACT TCA CTG GCC GCA GCT GAC TTG GTA GTG 816

Tn2
GGA CTC CTC GTA ATG CCA CCA GGG GCC ACA TTG GCG CTG ACT GGC CAT TGG CCC TTG GGC GAA ACT GGT TGC GAA 891

Tn3 Tn3
CTG TGG ACG TCA GTG GAC GTG CTC TGT GTA ACT GCT AGC ATC GAG ACC TTG TGC GCC CTG GCT GTG GAC CGC TAC 966

Tn4
CTA GCT GTC ACC AAC CCT TTG CGT TAC GGC ACG CTG GTT ACC AAG CGC CGC GCC CGC GCG GCA GTT GTC CTG GTG 1041

Tn4
TGG ATC GTG TCC GCT GCC GTG TCC TTT GCG CCC ATC ATG AGC CAG TGG TGG CGT GTA GGG GCA GAT GCC GAG GCA 1116

Tn5
CAG GAA TGC CAC TCC AAT CCG CGC TGC TGT TCC TTT GCC TCC AAC ATG CCC TAT GCG CTG CTC TCC TCC TCC GTC 1191

Tn5
TCC TTC TAC CTT CCC CTC CTT GTG ATG CTC TTC GTC TAT GCT CGA GTG TTC GTT GTG GCT AAG CGC CAA CGG CAT 1266

TTG CTG CGC CGG GAA CTG GGC CGC TTC TCG CCC GAG GAG TCT CCG CCG TCT CCG TCG CGC TCT CCG TCC CCT GCC 1341

ACA GGC GGG ACA CCC GCG GCA CCG GAT GGA GTG CCC CCC TGC GGC CGG CGG CCT GCG CGC CTC CTG CCA CTC CGG 1416

Tn6
GAA CAC CGC GCC CTG CGC ACC TTA GGT CTC ATT ATG GGC ATC TTC TCT CTG TGC TGG CTG CCC TTC TTC CTG GCC 1491

Tn6 Tn7
AAC GTG CTG CGC GCA CTC GCG GGG CCC TCT CTA GTT CCC AGC GGA GTT TTC ATC GCC CTG AAC TGG CTG GGC TAT 1566

Tn7
GCC AAC TCC GCC TTC AAC CCG GTC ATC TAC TGC CGC AGC CCG GAC TTT CGC GAC GCC TTC CGT CGT CTT CTG TGT 1641

AGC TAC GGT GGC CGT GGA CCG GAG GAG CCA CGC GCA GTC ACC TTC CCA GCC AGC CCT GTT GAA GCC AGG CAG AGT 1716

CCA CCG CTC AAC AGG TAG GGACACGAGCGGGGGACCGGAGTCTCTGGGTGGGGACGTCTCTGTCTCTATTTTTGAGTTTGGAGATTGGGGAG 1810

GGGAAGATGTAGATGGGGGTGCGGTGTGTGTGTGGGTGGGGGGTGGCCTTTGTCTTGAGAGGACAGAAAAGAGGTAGGAACTAAAACGGGCCCTTTCTCT 1910

TCTTGGATCC (I) 1920

2. Séquence selon la revendication 1, **caractérisée en ce qu'**elle comprend notamment les sites de restriction uniques suivants :
AlwN I, Sac I, Stu I, Dra I, Mse I, Bbe I, Nar I, Xcm I, NCo I, BspM I, Afl III, Pvu I, Nhe I, BstE II, BspH I, Bsm I, Nsp7524 I, NspH I, Xho I, Sac II, Eag I, Nae I, PaeR7 I, EcoN I, Fsp I, Bbv II, Nru I, Drd I, BamH I.

3. Fragment de la séquence selon la revendication 1 ou la revendication 2, **caractérisé en ce qu'**il est constitué d'un segment de 1164 paires de bases nucléotidiques, qui correspond aux nucléotides 568-1731 de la séquence de formule I.

4. Fragment de la séquence selon la revendication 1 ou la revendication 2, **caractérisé en ce qu'**il est constitué d'un segment de 1360 paires de bases contenant la région codante du gène β3 murin et une partie de la région 3' non traduite.

5. Fragment de la séquence selon la revendication 1 ou la revendication 2, **caractérisé en ce qu'**il est constitué d'un segment de 280 paires de bases nucléotidiques, qui correspond aux nucléotides 1640-1920 de la séquence de formule I.

6. Fragment de la séquence selon la revendication 1 ou la revendication 2, **caractérisé en ce qu'**il est constitué d'un segment de 608 paires de bases nucléotidiques, situé entre les régions transmembranaires TM3 et TM6 et qui correspond aux nucléotides 895-1503 de la séquence de formule I.

7. Fragment de la séquence selon la revendication 1 ou la revendication 2, **caractérisé en ce qu'**il est constitué d'un segment de 297 paires de bases nucléotidiques, allant du codon d'initiation de la traduction jusqu'à la fin du domaine TM2 et qui correspond aux nucléotides 567-864 (région du gène codant pour la partie amino-terminale du peptide) de la séquence de formule I.

8. Sondes nucléotidiques, **caractérisées en ce qu'**elles comprennent une séquence selon Tune quelconque des revendications 3 à 7 et **en ce qu'**elles sont éventuellement marquées à l'aide d'un marqueur tel qu'un isotope radioactif, une enzyme appropriée ou toute autre substance non radioactive appropriée.

9. Peptide et/ou fragment de celui-ci, **caractérisé en ce qu'**il est codé par une séquence nucléotidique selon Tune quelconque des revendications 1 à 4, et **en ce qu'**il présente une activité de récepteur β3-adrénergique, à savoir que, lorsque le fragment est exposé à la surface d'une cellule, il est capable de participer à l'activation de l'adénylate cyclase en présence d'un des agonistes suivants : BRL 28410, BRL 37344, CGP 12177A et (l)-isoprotérénol ; soit il est susceptible d'être reconnu par des anticorps qui ne reconnaissent ni le récepteur adrénergique β1, ni le récepteur adrénergique β2 ; soit il est susceptible de générer des anticorps qui ne reconnaissent ni le récepteur β1, ni le récepteur β2.

10. Peptide selon la revendication 9, **caractérisé en ce qu'**il comprend 388 amino-acides et présente la séquence en amino-acides de formule II suivante : 
Met-Ala-Pro-Trp-Pro-His-Arg-Asn-Gly-Ser-Leu-Ala-Leu-Trp-
Ser-Asp-Ala-Pro-Thr-Leu-Asp-Pro-Ser-Ala-Ala-Asn-Thr-Ser-
Gly-Leu-Pro-Gly-Val-Pro-Trp-Ala-Ala-Ala-Leu-Ala-Gly-Ala-
Leu-Leu-Ala-Leu-Ala-Thr-Val-Gly-Gly-Asn-Leu-Leu-Val-Ile-
Ile-Ala-Ile-Ala-Arg-Thr-Pro-Arg-Leu-Gln-Thr-Ile-Thr-Asn-
Val-Phe-Val-Thr-Ser-Leu-Ala-Ala-Ala-Asp-Leu-Val-Val-Gly-
Leu-Leu-Val-Met-Pro-Pro-Gly-Ala-Thr-Leu-Ala-Leu-Thr-Gly-
His-Trp-Pro-Leu-Gly-Glu-Thr-Gly-Cys-Glu-Leu-Trp-Thr-Ser-
Val-Asp-Val-Leu-Cys-Val-Thr-Ala-Ser-Ile-Glu-Thr-Leu-Cys-
Ala-Leu-Ala-Val-Asp-Arg-Tyr-Leu-Ala-Val-Thr-Asn-Pro-Leu-
Arg-Tyr-Gly-Thr-Leu-Val-Thr-Lys-Arg-Arg-Ala-Arg-Ala-Ala-
Val-Val-Leu-Val-Trp-Ile-Val-Ser-Ala-Ala-Val-Ser-Phe-Ala-
Pro-Ile-Met-Ser-Gln-Trp-Trp-Arg-Val-Gly-Ala-Asp-Ala-Glu-
Ala-Gln-Glu-Cys-His-Ser-Asn-Pro-Arg-Cys-Cys-Ser-Phe-Ala-
Ser-Asn-Met-Pro-Tyr-Ala-Leu-Leu-Ser-Ser-Ser-Val-Ser-Phe-
Tyr-Leu-Pro-Leu-Leu-Val-Met-Leu-Phe-Val-Tyr-Ala-Arg-Val-
Phe-Val-Val-Ala-Lys-Arg-Gln-Arg-His-Leu-Leu-Arg-Arg-Glu-
Leu-GLy-Arg-Phe-Ser-Pro-Glu-Glu-Ser-Pro-Pro-Ser-Pro-Ser-
 
Arg-Ser-Pro-Ser-Pro-Ala-Thr-Gly-Gly-Thr-Pro-Ala-Ala-Pro-
Asp-Gly-Val-Pro-Pro-Cys-Gly-Arg-Arg-Pro-Ala-Arg-Leu-Leu-
Pro-Leu-Arg-Glu-His-Arg-Ala-Leu-Arg-Thr-Leu-Gly-Leu-Ile-
Met-Gly-Ile-Phe-Ser-Leu-Cys-Trp-Leu-Pro-Phe-Phe-Leu-Ala-
Asn-Val-Leu-Arg-Ala-Leu-Ala-Gly-Pro-Ser-Leu-Val-Pro-Ser-
Gly-Val-Phe-Ile-Ala-Leu-Asn-Trp-Leu-Gly-Tyr-Ala-Asn-Ser-
Ala-Phe-Asn-Pro-Val-Ile-Tyr-Cys-Arg-Ser-Pro-Asp-Phe-Arg-
Asp-Ala-Phe-Arg-Arg-Leu-Leu-Cys-Ser-Tyr-Gly-Gly-Arg-Gly-
Pro-Glu-Glu-Pro-Arg-Ala-Val-Thr-Phe-Pro-Ala-Ser-Pro-Val-
Glu-Ala-Arg-Gln-Ser-Pro-Pro-Leu-Asn-Arg (II),
 lequel peptide présente 82 % d'homologie avec le peptide β3 humain.

11. Fragment du peptide selon la revendication 10, **caractérisé en ce qu'**il comprend un fragment de 25 amino-acides, correspondant à la région transmembranaire TMI de la séquence de formule II.

12. Fragment du peptide selon la revendication 10, **caractérisé en ce qu'**il comprend un fragment de 30 aminoacides, correspondant au fragment COOH terminal de la séquence de formule II.

13. Vecteur recombinant de clonage et/ou d'expression, **caractérisé en ce qu'**il comprend une séquence nucléotidique selon l'une quelconque des revendications 1 à 7.

14. Vecteur d'expression selon la revendication 13, **caractérisé en ce qu'**il est constitué par un vecteur recombinant approprié comprenant en particulier une origine de réplication dans un microorganisme hôte approprié, notamment une bactérie ou une cellule eucaryote, au moins un gène dont l'expression permet la sélection soit des bactéries, soit des cellules eucaryotes ayant reçu ledit vecteur, une séquence régulatrice appropriée, notamment un promoteur permettant l'expression des gènes dans lesdites bactéries ou cellules eucaryotes, et dans lequel est insérée une séquence nucléotidique selon Tune quelconque des revendications 1 à 7 ayant une activité de récepteur β3-adrénergique murin.

15. Vecteur selon la revendication 14, **caractérisé en ce qu'**il est constitué successivement d'une origine de réplication approprié, d'un gène de résistance à l'ampicilline, du promoteur viral SV40 d'une séquence nucléotidique selon l'une quelconque des revendications 1 à 4 codant pour un peptide ayant une activité de récepteur β3-adrénergique murin, d'un site de polyadénylation dérivé du virus de l'hépatite B (HBsAg) et du gène codant pour la dihydrofolate réductase (DHFR), enzyme qui permet la survie des cellules dans un milieu dépourvu en glycine, hypoxanthine et thymidine.

16. Vecteur selon la revendication 15, **caractérisé en ce qu'**il a été déposé sous le n° I-1026 en date du 14 janvier 1991 auprès de la Collection Nationale de Cultures de Microorganismes tenue par l'INSTITUT PASTEUR.

17. Cellule hôte appropriée, obtenue par transformation génétique, **caractérisée en ce qu'**elle est transformée par un vecteur d'expression selon l'une quelconque des revendications 13 à 16.

18. Cellule hôte selon la revendication 17, **caractérisée en ce qu'**elle est notamment constituée par les cellules de la lignée CHO (Chinese Hamster Ovary).

19. Processus d'expression d'un peptide selon l'une quelconque des revendications 9 à 12, **caractérisé en ce que** la cellule hôte, résultant de la transformation par un vecteur contenant une séquence nucléotidique, codant pour un peptide d'origine murine ayant une activité de récepteur β3-adrénergique, est cultivée de manière à produire et à transporter ledit peptide exprimé vers la membrane, de telle sorte que les séquences transmembranaires dudit récepteur β3 soient exposées à la surface de la membrane de l'hôte cellulaire transformé.

20. Modèle d'étude des récepteurs β3-adrénergiques, **caractérisé en ce qu'**il est constitué par des cellules hôtes selon l'une quelconque des revendications 17 et 18, c'est-à-dire exprimant un récepteur β3-adrénergique murin à la surface de leur membrane.

21. Procédé de détection de la capacité d'une substance à se comporter comme ligand vis-à-vis d'un peptide selon l'une quelconque des revendications 9 à 12, lequel procédé comprend :
- la mise en contact de ladite substance avec une cellule hôte préalablement transformée par un vecteur d'expression selon l'une quelconque des revendications 13 à 16, laquelle cellule hôte exprime ledit peptide murin (RAβ3 murin), le cas échéant après induction physique ou chimique appropriée, et laquelle mise en contact est réalisée dans des conditions permettant la formation d'une liaison entre l'un au moins des sites spécifiques et ladite substance s'il y a lieu et
- la détection de la formation éventuelle d'un complexe du type ligand-peptide.

22. Méthode de production de souris recombinantes, n'exprimant plus le récepteur β3-adrénergique, **caractérisée en ce que** :
- l'on réalise, dans la séquence de formule I, selon la revendication 1, une mutation convenable qui inhibe l'expression du gène codant pour le récepteur β3-adrénergique murin ;
- l'on introduit ladite séquence I modifiée dans un segment de l'AD génomique de souris, associé à un marqueur approprié, de manière à obtenir une séquence marquée M, contenant la séquence de formule I modifiée ;
- l'on intègre ladite séquence *M, in vitro,* dans les cellules souches de lignées germinales d'embryon de souris ; puis
- l'on réinjecte lesdites cellules souches à une souris ; et après recombinaison homologue,
- l'on obtient à la génération F2 des souris recombinées homozygotes, reconnaissables à la présence du marqueur.

23. Méthode selon la revendication 22, **caractérisée en ce que** ledit segment d'ADN génomique de souris comprend au plus 14 Kbases.

24. Méthode selon la revendication 23, **caractérisée en ce que** lorsque ledit fragment est de 14 Kbases, il comprend notamment les sites de restriction suivants : 3 sites SphI, 2 sites BamHI, 2 sites HindIII, 4 sites XbaI, 4 sites SacI, 1 site KpnI, 1 site SmaI, 2 sites BglII, 2 sites PvuII, 1 site PstI et 1 site EcoRI.

25. Méthode selon Tune quelconque des revendications 22 à 24, **caractérisée en ce que** la séquence de formule I mutée est comprise entre un site BglII et un site BamHI du segment d'ADN génomique de souris.

26. Procédé pour l'étude de l'affinité d'un peptide selon l'une quelconque des revendications 9 à 12 pour un ou plusieurs ligands déterminés, lequel procédé comprend :
- la transformation d'une cellule hôte appropriée par un vecteur d'expression selon Tune quelconque des revendications 13 à 16 ;
- la culture de la cellule hôte transformée, dans des conditions permettant l'expression du récepteur β3 codée par la séquence nucléotidique, et le transfert du récepteur β3 exprimé vers la membrane de ladite cellule, de sorte que les séquences transmembranaires du récepteur β3 soient exposées à la surface de la cellule hôte transformée ;
- la mise en contact de ladite cellule avec les ligands déterminés ; et
- la détection d'une réaction affine entre ladite cellule transformée et lesdits ligands déterminés.

27. Kit ou trousse de détection de l'affinité éventuelle d'un ligand pour un peptide selon l'une quelconque des revendications 9 à 12, lequel kit comprend :
- une culture de cellules hôtes transformées par un vecteur d'expression selon l'une quelconque des revendications 13 à 16 ;
- des moyens physiques ou chimiques pour induire l'expression d'un peptide (récepteur β3 murin ou un fragment de celui-ci) codé par une séquence nucléotidique selon l'une quelconque des revendications 1 à 4, contenue dans un vecteur dont le promoteur est inductible ;
- un ou plusieurs ligands témoins ayant des affinités déterminées pour ledit peptide ; et
- des moyens physiques ou chimiques pour la caractérisation de l'activité biologique du peptide exprimé.

28. Anticorps dirigés de façon spécifique contre l'un des peptides selon l'une quelconques 9 à 12, **caractérisés en ce qu'**ils ne reconnaissent ni le récepteur adrénergique β1, ni le récepteur adrénergique β2.

29. Anticorps selon la revendication 28, **caractérisé en ce qu'**ils sont des anticorps polyclonaux.

30. Anticorps selon la revendication 28, **caractérisé en ce qu'**ils sont des anticorps monoclonaux.

## Claims

1. Nucleic acid sequence, **characterised in that** it comprises the murine gene coding for a peptide having a β3-adrenegenic receptor activity, and **in that** it includes 1920 base pairs and comprises the nucleotide sequence of Formula I below:

GATCTGTAATCCCAGCACTGGGGAGGTTGAGGCAGAAGGATCTGGAGGTCCAGACCAATCTGGGCAACATATAGAAAGACTATCTCAAACAATAAGATAC 100

CTTAGGGAGAGCATCCAAGCAGAAGAGGGGCTATCTTGGATGGTTTGGGTTGTTCGGTTTTGTTTTGGTTTGTTTCTGGATGGTTGCCTTCCTTGTTGGG 200

TAAAGGATAGGGTGCGGGGGTTTCTCTTCTTTGCAGGGTTGCCTCAGGTTCTGCCAGGAAGGAGCTGCTGAGCTCCAGGAAACCGGTGCTGAGGGAGTGT 300

CAAGACAGGACGCCCCTCTCCACCCTCCAATTCCCACCAGAGGCCTCTCTTGTGACTATTGGACGCTGTTCGTTTAAAAGCAGCCACTCCTCCGGGCAAC 400

TAGGGTGTACATGGGGGGTGAGATGGAGGGAAGCTGACAGACTTACCCCAGCAATTAGGGAAGATGGCCCCAGGCTGGAAGAGTCGGCTCCCAAGCCCTACT 500

GTCCCCTTCCCTAAGCCAGCGGGTCTGGGGAGGAGGGGAACCTTCCCACCCCAGGCGCCACACGAG ATG GCT CCC TGC CCT CAC AGA AAC 591

GGC TCT CTG GCT TTG TGG TCG GAC GCC CCT ACC CTG GAC CCC AGT GCA GCC AAC ACC AGT GGG TTG CCA GGA GTA 666

TM1
CCA TGG GCA GCG GCA TTG GCT GGG GCA TTG CTG GCG CTG GCC ACG GTG GGA GGC AAC CTG CTG GTA ATC ATA GCC 741

TM1 ← →TM2
ATC GCC CGC ACG CCG AGA CTA CAG ACC ATA ACC AAC GTG TTC GTG ACT TCA CTG GCC GCA GCT GAC TTG GTA GTG 816

TM2 ←
GGA CTC CTC GTA ATG CCA CCA GGG GCC ACA TTG GCG CTG ACT GGC CAT TGG CCC TTG GGC GAA ACT GGT TGC GAA 891

→TM3 TM3 ←
CTG TGG ACG TCA GTG GAC GTG CTC TGT GTA ACT GCT AGC ATC GAG ACC TTG TGC GCC CTG GCT GTG GAC CGC TAC 966

→TM4
CTA GCT GTC ACC AAC CCT TTG CGT TAC GGC ACG CTG GTT ACC AAG CGC CGC GCC CGC GCG GCA GTT GTC CTG GTG 1041

TM4 ←
TGG ATC GTC TCC GCT GCC GTG TCC TTT GCG CCC ATC ATG AGC CAG TGG TGG CGT GTA GGG GCA GAT GCC GAG GCA 1116

→TM5
CAG GAA TGC CAC TCC AAT CCG CGC TGC TGT TCC TTT GCC TCC AAC ATG CCC TAT GCG CTG CTC TCC TCC TCC GTC 1191

TM5 ←
TCC TTC TAC CTT CCC CTC CTT GTG ATG CTC TTC GTC TAT GCT CGA GTG TTC GTT GTG GCT AAG CGC CAA CGG CAT 1266

TTG CTG CGC CGG GAA CTG GGC CGC TTC TCG CCC GAG GAG TCT CCG CCG TCT CCG TCG CGC TCT CCG TCC CCT GCC 1341

ACA GGC GGG ACA CCC GCG GCA CCG GAT GGA GTG CCC CCC TGC GGC CGG CGG CCT GCG CGC CTC CTG CCA CTC CGG 1416

→TM6
GAA CAC CGC GCC CTG CGC ACC TTA GGT CTC ATT ATG GGC ATC TTC TCT CTG TCC TGG CTG CCC TTC TTC CTG GCC 1491

TM6 ← →TM7
AAC GTG CTG CGC GCA CTC GCG GGG CCC TCT CTA GTT CCC AGC GGA GTT TTC ATC GCC CTG AAC TGG CTG GGC TAT 1566

TM7 ←
GCC AAC TCC GCC TTC AAC CCG GTC ATC TAC TGC CGC AGC CCG GAC TTT CGC GAC GCC TTC CGT CGT CTT CTG TGT 1641

AGC TAC GGT GGC CGT GGA CCG GAG GAG CCA CGC GCA GTC ACC TTC CCA GCC AGC CCT GTT GAA GCC AGG CAG AGT 1716

CCA CCG CTC AAC AGG TAG GGGACACGAGCGGGGGACCGGAGTCTCTGGGTGGGGACGTCTCTGTCTCTATTTTTGAGTTTGGAGATTGGGGGAG 1810

GGGAAGATGTAGATGGGGGTGCGGTGTGTGTGTGGGTGGGGGGTGGCCTTTTGTCTTGAGAGGACAGAAAAGAGGTAGGAACTAAAACGGGCCCTTTTCTCT 1910

TCTTGGATCC (I) 1920

2. Sequence according to Claim 1, **characterised in that** it comprises, in particular, the following unique restriction sites:
AlwN I, Sac I, Stu I, Dra I, Mse I, Bbe I, Nar I, Xcm I, NCO I, BspM I, Afl III, Pvu I, Nhe I, BstE II, BspH I, Bsm I, Nsp7524 I, NspH I, Xho I, Sac II, Eag I, Nae I, PaeR7 I, EcoN I, Fsp I, Bbv II, Nru I, Drd I, BamH I.

3. Fragment of the sequence according to Claim 1 or Claim 2, **characterised in that** it consists of a segment with 1164 nucleotide base pairs, which corresponds to the nucleotides 568-1731 of the sequence of Formula I.

4. Fragment of the sequence according to Claim 1 or Claim 2, **characterised in that** it consists of a segment with 1360 base pairs containing the region coding for the murine β3 gene and a portion of the 3' untranslated region.

5. Fragment of the sequence according to Claim 1 or Claim 2, **characterised in that** it consists of a segment with 280 nucleotide base pairs, which corresponds to the nucleotides 1640-1920 of the sequence of Formula I.

6. Fragment of the sequence according to Claim 1 or Claim 2, **characterised in that** it consists of a segment with 608 nucleotide base pairs, which is located between the TM3 and TM6 transmembrane regions and which corresponds to the nucleotides 895-1503 of the sequence of Formula I.

7. Fragment of the sequence according to Claim 1 or Claim 2, **characterised in that** it consists of a segment with 297 nucleotide base pairs, which extends from the translation start codon to the end of the TM2 domain and which corresponds to the nucleotides 567-864 (region of the gene coding for the amino-terminal portion of the peptide) of the sequence of Formula I.

8. Nucleotide probes, **characterised in that** they comprise a sequence according to any one of Claims 3 to 7, and **in that** they are optionally labelled by means of a marker such as a radioactive isotope, a suitable enzyme or any other suitable non-radioactive substance.

9. Peptide and/or fragment thereof, **characterised in that** it is encoded by a nucleotide sequence according to any one of Claims 1 to 4, and **in that** it has a β3-adrenegenic receptor activity, i.e. when the fragment is exposed to the surface of a cell, it is capable of participating in the activation of adenylate cyclase in the presence of one of the following agonists: BRL 28410, BRL 37344, CGP 12177A and (1)-isoproterenol; either it is capable of being recognised by antibodies which recognise neither the β1 adrenegenic receptor nor the β2 adrenegenic receptor; or it is capable of generating antibodies which recognise neither the β1 receptor nor the β2 receptor.

10. Peptide according to Claim 9, **characterised in that** it comprises 388 amino acids and has the amino acid sequence of the following Formula II: 
Met-Ala-Pro-Trp-Pro-His-Arg-Asn-Gly-Ser-Leu-Ala-Leu-Trp-
Ser-Asp-Ala-Pro-Thr-Leu-Asp-Pro-Ser-Ala-Ala-Asn-Thr-Ser-
Gly-Leu-Pro-Gly-Val-Pro-Trp-Ala-Ala-Ala-Leu-Ala-Gly-Ala-
Leu-Leu-Ala-Leu-Ala-Thr-Val-Gly-Gly-Asn-Leu-Leu-Val-Ile-
 
Ile-Ala-Ile-Ala-Arg-Thr-Pro-Arg-Leu-Gln-Thr-Ile-Thr-Asn-
Val-Phe-Val-Thr-Ser-Leu-Ala-Ala-Ala-Asp-Leu-Val-Val-Gly-
Leu-Leu-Val-Met-Pro-Pro-Gly-Ala-Thr-Leu-Ala-Leu-Thr-Gly-
His-Trp-Pro-Leu-Gly-Glu-Thr-Gly-Cys-Glu-Leu-Trp-Thr-Ser-
Val-Asp-Val-Leu-Cys-Val-Thr-Ala-Ser-Ile-Glu-Thr-Leu-Cys-
Ala-Leu-Ala-Val-Asp-Arg-Tyr-Leu-Ala-Val-Thr-Asn-Pro-Leu-
Arg-Tyr-Gly-Thr-Leu-Val-Thr-Lys-Arg-Arg-Ala-Arg-Ala-Ala-
Val-Val-Leu-Val-Trp-Ile-Val-Ser-Ala-Ala-Val-Ser-Phe-Ala-
Pro-Ile-Met-Ser-Gln-Trp-Trp-Arg-Val-Gly-Ala-Asp-Ala-Glu-
Ala-Gln-Glu-Cys-His-Ser-Asn-Pro-Arg-Cys-Cys-Ser-Phe-Ala-
Ser-Asn-Met-Pro-Tyr-Ala-Leu-Leu-Ser-Ser-Ser-Val-Ser-Phe-
Tyr-Leu-Pro-Leu-Leu-Val-Met-Leu-Phe-Val-Tyr-Ala-Arg-Val-
Phe-Val-Val-Ala-Lys-Arg-Gln-Arg-His-Leu-Leu-Arg-Arg-Glu-
Leu-Gly-Arg-Phe-Ser-Pro-Glu-Glu-Ser-Pro-Pro-Ser-Pro-Ser-
Arg-Ser-Pro-Ser-Pro-Ala-Thr-Gly-Gly-Thr-Pro-Ala-Ala-Pro-
Asp-Gly-Val-Pro-Pro-Cys-Gly-Arg-Arg-Pro-Ala-Arg-Leu-Leu-
Pro-Leu-Arg-Glu-His-Arg-Ala-Leu-Arg-Thr-Leu-Gly-Leu-Ile-
Met-Gly-Ile-Phe-Ser-Leu-Cys-Trp-Leu-Pro-Phe-Phe-Leu-Ala-
Asn-Val-Leu-Arg-Ala-Leu-Ala-Gly-Pro-Ser-Leu-Val-Pro-Ser-
Gly-Val-Phe-Ile-Ala-Leu-Asn-Trp-Leu-Gly-Tyr-Ala-Asn-Ser-
Ala-Phe-Asn-Pro-Val-Ile-Tyr-Cys-Arg-Ser-Pro-Asp-Phe-Arg-
Asp-Ala-Phe-Arg-Arg-Leu-Leu-Cys-Ser-Tyr-Gly-Gly-Arg-Gly-
Pro-Glu-Glu-Pro-Arg-Ala-Val-Thr-Phe-Pro-Ala-Ser-Pro-Val-
Glu-Ala-Arg-Gln-Ser-Pro-Pro-Leu-Asn-Arg     (II),
 which peptide has 82% homology with the human β3 peptide.

11. Fragment of the peptide according to Claim 10, **characterised in that** it comprises a fragment with 25 amino acids, corresponding to the TMI transmembrane region of the sequence of Formula II.

12. Fragment of the peptide according to Claim 10, **characterised in that** it comprises a fragment with 30 amino acids, corresponding to the COOH terminal fragment of the sequence of Formula II.

13. Recombinant cloning and/or expression vector, **characterised in that** it comprises a nucleotide sequence according to any one of Claims 1 to 7.

14. Expression vector according to Claim 13, **characterised in that** it consists of a suitable recombinant vector comprising in particular an origin of replication in a suitable host microorganism, especially a bacterium or a eukaryotic cell, at least one gene whose expression allows the selection either of bacteria or of eukaryotic cells which have received said vector, a suitable regulatory sequence, especially a promoter which permits the expression of genes in said bacteria or eukaryotic cells, and in which a nucleotide sequence according to any one of Claims 1 to 7 having a murine β3-adrenergic receptor activity is inserted.

15. Vector according to Claim 14, **characterised in that** it successively consists of a suitable replication origin, a gene for resistance to ampicillin, the viral promoter SV40 [comma omitted] a nucleotide sequence according to any one of Claims 1 to 4 which codes for a peptide having a murine β3-adrenergic receptor activity, a polyadenylation site derived from the hepatitis B virus (HBsAg) and the gene which codes for dihydrofolate reductase (DHFR), which enzyme permits the survival of cells in a medium lacking glycine, hypoxanthine and thymidine.

16. Vector according to Claim 15, **characterised in that** it was filed under No I-1026 dated 14 January 1991 at the Collection Nationale de Cultures de Microorganismes held by the INSTITUT PASTEUR.

17. Suitable host cell, obtained by genetic transformation, **characterised in that** it is transformed by an expression vector conforming to the invention according to any one of Claims 13 to 16.

18. Host cell according to Claim 17, **characterised in that** it especially consists of cells of the CHO (Chinese Hamster Ovary) line.

19. Procedure for expressing a peptide according to any one of Claims 9 to 12, **characterised in that** the host cell, resulting from transformation by a vector containing a nucleotide sequence which codes for a peptide of murine origin having a β3-adrenergic receptor activity, is cultured so as to produce and transport said expressed peptide to the membrane, such that the transmembrane sequences of said β3 receptor are exposed at the surface of the membrane of the transformed host cell.

20. Model for studying the β3-adrenergic receptors, **characterised in that** it consists of host cells according to either one of Claims 17 and 18, that is to say expressing a murine β3-adrenergic receptor at the surface of their membrane.

21. Process for detecting the capacity of a substance to behave as a ligand with respect to a peptide according to any one of Claims 9 to 12, which process comprises:
- bringing said substance into contact with a host cell previously transformed by an expression vector according to any one of Claims 13 to 16, which host cell expresses said murine peptide (murine RAβ3), where appropriate after suitable physical or chemical induction, and contact is carried out under conditions which permit the formation of a bond between at least one of the specific sites and said substance if applicable and
- detecting the formation, if any, of a complex of the ligand-peptide type.

22. Method for producing recombinant mice, no longer expressing the β3-adrenergic receptor, **characterised in that**:
- a suitable mutation, which inhibits expression of the gene coding for the murine β3-adrenergic receptor, is produced in the sequence of Formula I according to Claim 1;
- said modified sequence I is introduced into a mouse genomic DNA segment, combined with a suitable marker, so as to obtain a labelled sequence M containing the modified sequence of Formula I;
- said sequence M is integrated in *vitro* into the stem cells of mouse embryo germ lines; then
- said stem cells are re-injected into a mouse; and after homologous recombination,
- homozygotic recombinant mice, which are recognisable by the presence of the marker, are obtained in the F2 generation.

23. Method according to Claim 22, **characterised in that** said mouse genomic DNA segment comprises at most 14 Kbases.

24. Method according to Claim 23, **characterised in that**, when said fragment has 14 Kbases, it comprises especially the following restriction sites: 3 SphI sites, 2 BamHI sites, 2 HindIII sites, 4 XbaI sites, 4 SacI sites, 1 KpnI site, 1 SmaI site, 2 BglII sites, 2 PvuII sites, 1 PstI site and 1 EcoRI site.

25. Method according to any one of Claims 22 to 24, **characterised in that** the mutated sequence of Formula I is contained between a BglII site and a BamHI site of the mouse genomic DNA segment.

26. Process for studying the affinity of a peptide according to any one of Claims 9 to 12 for one or more determined ligands, which process comprises:
- transforming a suitable host cell by an expression vector according to any one of Claims 13 to 16;
- culturing the transformed host cell under conditions which permit expression of the β3 receptor encoded by the nucleotide sequence, and transferring the expressed β3 receptor to the membrane of said cell, so that the transmembrane sequences of the β3 receptor are exposed at the surface of the transformed host cell;
- bringing said cell into contact with the determined ligands; and
- detecting an affinity reaction between said transformed cell and said determined ligands.

27. Kit or assembly for detecting the affinity, if any, of a ligand for a peptide according to any one of Claims 9 to 12, which kit comprises:
- a culture of host cells transformed by an expression vector according to any one of Claims 13 to 16;
- physical or chemical means for inducing the expression of a peptide (murine β3 receptor or a fragment thereof) encoded by a nucleotide sequence according to any one of Claims 1 to 4, which is contained in a vector whose promoter is inducible;
- one or more control ligands having determined affinities for said peptide; and
- physical or chemical means for **characterising** the biological activity of the peptide which is expressed.

28. Antibodies directed specifically against one of the peptides according to any one of Claims 9 to 12, **characterised in that** they recognise neither the β1 adrenergic receptor nor the β2 adrenergic receptor.

29. Antibodies according to Claim 28, **characterised in that** they are polyclonal antibodies.

30. Antibodies according to Claim 28, **characterised in that** they are monoclonal antibodies.

## Patentansprüche

1. Nukleinsäuresequenz, **dadurch gekennzeichnet, dass** sie das murine Gen umfasst, das für ein Deptid, das β3-Adrenozeptor-Aktivität hat, codiert und dass sie 1920 Basenpaare enthält und die Nukleotidsequenz der folgenden Formel (I) umfasst:

GATCTGTAATCCCAGCACTGGGGAGGTTGAGGCAGAAGGATCTGGAGGTCCAGACCAATCTGGGCAACATATAGAAAGACTATCTCAAACAATAAGATAC 100

CTTAGGGAGAGCATCCAAGCAGAAGAGGGGCTATCTTGGATGGTTTGGGTTGTTGGGTTTTGTTTTGGTTTGTTTCTGGATGGTTGGCTTCCTTGTTGGG 200

TAAAGGATAGGGTGCGGGGGCTTTCTCTTCTTTGCAGGGTTGCCTCAGGTTCTGCCAGGAAGGAGCTGCTGAGCTCCAGGAAACCGGTGCTGAGGGAGTGT 300

CAAGACAGGACGCCCCTCTGGACCCTCCAATTCCCACCAGAGCCCTCTCTTGTGACTATTGGACGCTGTTCCTTTAAAAGCAGCCACTCCTCCCGGCAAC 400

TAGGGTGTACATGGGGGGTGAGATGGAGGGAAGCTGACAGACTTACCCCAGCAATTAGGGAAGATGGCCCAGGCTGGAAGAGTCGCTCCCAAGCCCTACT 500

GTCCCCTTCCCTAAGCCAGCGGGTCTGGGGAGGAGGGGGAACCTTCCCACCCCAGGCGGCACACCAG ATG GCT CCC TGG CCT CAC AGA AAC 591

GGC TCT CTG GCT TTG TGG TCG GAC GCC CCT ACC CTG GAC CCC AGT GCA GCC AAC ACC AGT GGG TTG CCA GGA GTA 666

TM1
CCA TGG GCA GCG GCA TTG GCT GGG GCA TTG CTG GCG CTG GCC ACG GTG GGA GGC AAC CTG CTG GTA ATC ATA GCC 741

TM1 TM2
ATC GCC CGC ACG CCG AGA CTA CAG ACC ATA ACC AAC GTG TTC GTG ACT TCA CTG GCC GCA GCT GAC TTG GTA GTG 816

TM2
GGA CTC CTC GTA ATG CCA CCA GGG GCC ACA TTG GCG CTG ACT GGC CAT TGG CCC TTG GGC GAA ACT GGT TGC GAA 891

TM3 TM3
CTG TGG ACG TCA GTG GAC GTG CTC TGT GTA ACT GCT AGC ATC GAG ACC TTG TGC GCC CTG GCT GTG GAC CGC TAC 966

TM4
CTA GCT GTC ACC AAC CCT TTG CGT TAC GGC ACG CTG GTT ACC AAG CGC CGC GCC CGC GCG GCA GTT GTC CTG GTG 1041

TM4
TGG ATC GTG TCC GCT GCC GTG TCC TTT GCG CCC ATC ATG AGC CAG TGG TGG CGT GTA GGG GCA GAT GCC GAG GCA 1116

TM5
CAG GAA TGC CAC TCC AAT CCG CGC TGC TGT TCC TTT GCC TCC AAC ATG CCC TAT GCG CTG CTC TCC TCC TCC GTC 1191

TM5
TCC TTC TAC CTT CCC CTC CTT GTG ATG CTC TTC GTC TAT GCT CGA GTG TTC GTT GTG GCT AAG CGC CAA CGG CAT 1266

TTG CTG CGC CGG GAA CTG GGC CGC TTC TCG CCC GAG GAG TCT CCG CCG TCT CCG TCG CGC TCT CCG TCC CCT GCC 1341

ACA GGC GGG ACA CCC GCG GCA CCG GAT GGA GTG CCC CCC TGC GGC CGG CGG CCT GCG CGC CTC CTG CCA CTC CGG 1416

TM6
GAA CAC CGC GCC CTG CGC ACC TTA GGT CTC ATT ATC GGC ATC TTC TCT CTG TGC TGG CTG CCC TTC TTC CTG GCC 1491

TM6 TM7
AAC GTG CTG CGC GCA CTC GCG GGG CCC TCT CTA GTT CCC AGC GGA GTT TTC ATC GCC CTG AAC TGG CTG GGC TAT 1566

TM7 [?]

GCC AAC TCC GCC TTC AAC CCG GTC ATC TAC TGC CGC AGC CCG GAC TTT CGC GAC GCC TTC CGT CGT CTT CTG TGT 1641

AGC TAC GGT GGC CGT GGA CCG GAG GAG CCA CGC GCA GTC ACC TTC CCA GCC AGC CCT GTT GAA GCC AGG CAG AGT 1716

CCA CCG CTC AAC AGG TAG GGGACACCGAGCGGGGGACCCGAGTCTCTGGCTGGGCACGTCTCTGTCTCTATTTTTGAGTTTGGAGATTGGGGAG 1810

GGCAAGATGTAGATGGGGGTGCGGTGTGTGTGTGGGTGGGGGGTGGCCTTTGTCTTGAGAGGACAGAAAAGAGGTAGGAACTAAAACGGGCCCCTTTCTCT 1910

TCTTGGATCC 1920

(I)

2. Sequenz nach Anspruch 1, **dadurch gekennzeichnet, dass** sie insbesondere die folgenden nur einmal vorhandenen Restriktions-Schnittstellen umfasst: AlwN I, Sac I, Stu I, Dra I, Mse I, Bbe I, Nar I, Xcm I, Nco I, BspM I, Afl III, Pvu I, Nhe I, BstE II, BspH I, Bsm I, Nsp7524 I, NspH I, Xho I, Sac II, Eag I, Nae I, PaeR7 I, EcoN I, Fsp I, Bbv II, Nru I, Drd I, BamH I.

3. Fragment der Sequenz nach Anspruch 1 oder Anspruch 2, **dadurch gekennzeichnet, dass** es aus einem Segment mit 1164 Nukleotidbasenpaaren bestaht, das den Nukleotiden 568-1731 der Sequenz der Formel I entspricht.

4. Fragment der Sequenz nach Anspruch 1 oder Anspruch 2, **dadurch gekennzeichnet, dass** es aus einem Segment mit 1360 Basenpaaren besteht, das die Region, die für das murine Gen β3 codiert, und einen Teil der nichttranslatierten 3'-Region enthält.

5. Fragment der Sequenz nach Anspruch 1 oder Anspruch 2', **dadurch gekennzeichnet, dass** es aus einem Segment mit 280 Nukleotidbasenpaaren besteht, das den Nukleotiden 1640-1920 der Sequenz der Formel I entspricht.

6. Fragment der Sequenz nach Anspruch 1 oder Anspruch 2, **dadurch gekennzeichnet, dass** es aus einem Segment mit 608 Nukleotidbasenpaaren besteht, das zwischen den Transmembranregionen TM3 und TM6 liegt und das den Nukleotiden 895-1503 der Sequenz der Formel I entspricht.

7. Fragment der Sequenz nach Anspruch 1 oder Anspruch 2, **dadurch gekennzeichnet, dass** es aus einem Segment mit 297 Nukleotidbasenpaaren besteht, das vom Initiati-onscodon der Translation bis zum Ende der Domäne TM2 geht und den Nukleotiden 567-864 (Region des Gens, das für den aminoterminalen Teil des Peptids codiert) der Sequenz der Formel I entspricht.

8. Nukleotidsonden, **dadurch gekennzeichnet, dass** sie eine Sequenz nach einem der Ansprüche 3 bis 7 umfassen und dass sie ggf. mit Hilfe eines Markers, z.B. ein radioaktives Isotop, ein geeignetes Enzym oder jede andere geeignete, nicht-radioaktive Substanz, markiert sind.

9. Peptid und/oder Fragment desselben, **dadurch gekennzeichnet, dass** es durch eine Nukleotidsequenz nach einem der Ansprüche 1 bis 4 codiert ist und dass es β3-Adrenozeptor-Aktivität hat, das, wenn das Fragment an der Oberfläche einer Zelle exponiert ist, fähig ist, an der Aktivierung der Adenelatzyklase in Gegenwart eines der folgenden Agonisten BRL 28410, BRL 37344, CGP 12177A und (1)-Isoproterenol teilzunehmen oder von Antikörpern erkannt werden kann, die weder den β1-Adrenozeptor noch den β2-Adrenozeptor erkennen, oder Antikörper erzeugen kann, die weder den Rezeptor β1 noch den Rezeptor β2 erkennen.

10. Peptid nach Anspruch 9, **dadurch gekennzeichnet, dass** es 388 Aminosäuren umfasst und die Aminosäuresequenz der folgenden Formel II zeigt: 
Met-Ala-Pro-Trp-Pro-His-Arg-Asn-Gly-Ser-Leu-Ala-Leu-Trp-
Ser-Asp-Ala-Pro-Thr-Leu-Asp-Pro-Ser-Ala-Ala-Asn-Thr-Ser-
Gly-Leu-Pro-Gly-Val-Pro-Trp-Ala-Ala-Ala-Leu-Ala-Gly-Ala-
Leu-Leu-Ala-Leu-Ala-Thr-Val-Gly-Gly-Asn-Leu-Leu-Val-Ile-
Ile-Ala-Ile-Ala-Arg-Thr-Pro-Arg-Leu-Gln-Thr-Ile-Thr-Asn-
Val-Phe-Val-Thr-Ser-Leu-Ala-Ala-Ala-Asp-Leu-Val-Val-Gly-
 
Leu-Leu-Val-Met-Pro-Pro-Gly-Ala-Thr-Leu-Ala-Leu-Thr-Gly-
His-Trp-Pro-Leu-Gly-Glu-Thr-Gly-Cys-Glu-Leu-Trp-Thr-Ser-
Val-Asp-Val-Leu-Cys-Val-Thr-Ala-Ser-Ile-Glu-Thr-Leu-Cys-
Ala-Leu-Ala-Val-Asp-Arg-Tyr-Leu-Ala-Val-Thr-Asn-Pro-Leu-
Arg-Tyr-Gly-Thr-Leu-Val-Thr-Lys-Arg-Arg-Ala-Arg-Ala-Ala-
Val-Val-Leu-Val-Trp-Ile-Val-Ser-Ala-Ala-Val-Ser-Phe-Ala-
Pro-Ile-Met-Ser-Gln-Trp-Trp-Arg-Val-Gly-Ala-Asp-Ala-Glu-
Ala-Gln-Glu-Cys-His-Ser-Asn-Pro-Arg-Cys-Cys-Ser-Phe-Ala-
Ser-Asn-Met-Pro-Tyr-Ala-Leu-Leu-Ser-Ser-Ser-Val-Ser-Phe-
Tyr-Leu-Pro-Leu-Leu-Val-Met-Leu-Phe-Val-Tyr-Ala-Arg-Val-
Phe-Val-Val-Ala-Lys-Arg-Gln-Arg-His-Leu-Leu-Arg-Arg-Glu-
Leu-GLy-Arg-Phe-Ser-Pro-Glu-Glu-Ser-Pro-Pro-Ser-Pro-Ser-
Arg-Ser-Pro-Ser-Pro-Ala-Thr-Gly-Gly-Thr-Pro-Ala-Ala-Pro-
Asp-Gly-Val-Pro-Pro-Cys-Gly-Arg-Arg-Pro-Ala-Arg-Leu-Leu-
Pro-Leu-Arg-Glu-His-Arg-Ala-Leu-Arg-Thr-Leu-Gly-Leu-Ile-
Met-Gly-Ile-Phe-Ser-Leu-Cys-Trp-Leu-Pro-Phe-Phe-Leu-Ala-
Asn-Val-Leu-Arg-Ala-Leu-Ala-Gly-Pro-Ser-Leu-Val-Pro-Ser-
Gly-Val-Phe-Ile-Ala-Leu-Asn-Trp-Leu-Gly-Tyr-Ala-Asn-Ser-
Ala-Phe-Asn-Pro-Val-Ile-Tyr-Cys-Arg-Ser-Pro-Asp-Phe-Arg-
Asp-Ala-Phe-Arg-Arg-Leu-Leu-Cys-Ser-Tyr-Gly-Gly-Arg-Gly-
Pro-Glu-Glu-Pro-Arg-Ala-Val-Thr-Phe-Pro-Ala-Ser-Pro-Val-
Glu-Ala-Arg-Gln-Ser-Pro-Pro-Leu-Asn-Arg (II),
 wobei das Peptid eine 82%ige Homologie zu dem humanen Peptid β3 zeigt.

11. Fragment des Peptids nach Anspruch 10, **dadurch gekennzeichnet, dass** es ein Peptid mit 25 Aminosäuren umfasst, das der Transmembranregion TMI der Sequenz der Formel II entspricht.

12. Fragment des Peptids nach Anspruch 10, **dadurch gekennzeichnet, dass** es ein Fragment mit 30 Aminosäuren umfasst, das dem COOH-terminalen Fragment der Sequenz der Formel II entspricht.

13. Rekombinanter Klonierungs- und/oder Expressionsvektor, **dadurch gekennzeichnet, dass** er eine Nukleotidsequenz nach einem der Ansprüche 1 bis 7 umfasst.

14. Expressionsvektor nach Anspruch 13, **dadurch gekennzeichnet, dass** er aus einem geeigneten rekombinanten Vektor besteht, der insbesondere einen Replikationsursprung in einem geeigneten Wirtsorganismus, insbesondere ein Bakterium oder eine eukaryontische Zelle, mindestens ein Gen, dessen Expression die Selektion der Bakterien oder der eukaryontischen Zellen, die den Vektor aufgenommen haben, zulässt, eine geeignete Regulationssequenz, insbesondere einen Promotor, der die Expression der Gene in den Bakterien oder eukaryontischen Zellen ermöglicht, umfasst und in den eine Nukleotidsequenz nach einem der Ansprüche 1 bis 7, die zu β3-Adrenozeptor-Aktivität führt, insertiert ist.

15. Vektor nach Anspruch 14, **dadurch gekennzeichnet, dass** er der Reihe nach aus einem geeigneten Replikationsursprung, einem Gen für Ampicillinresistenz, dem viralen Promotor SV40, einer Nukleotidsequenz nach einem der Ansprüche 1 bis 4, die für ein Peptid mit muriner β3-Adrenozeptor-Atkivität codiert, einer Polyadenylierungsstelle, die vom Hepatitis B-virus (HbsAg) stammt und dem Gen, das für die Dihydrofolatreduktase (DHFR), ein Enzym, das das Überleben von Zellen in einem Milieu ohne Glycin, Hypoxantin und Thymidin ermöglicht, codiert, besteht.

16. Vektor nach Anspruch 15, **dadurch gekennzeichnet, dass** er am 14. Januar 1991 unter der Nummer I-1026 bei der Collection Nationale de Culture de Microorganismes, geführt vom INSTITUT PASTEUR, hinterlegt wurde.

17. Geeignete Wirtszelle, die durch genetische Transformation erhalten wird, **dadurch gekennzeichnet, dass** sie durch den Expressionsvektor nach einem der Ansprüche 13 bis 16 transformiert ist.

18. Wirtszelle nach Anspruch 17, **dadurch gekennzeichnet, dass** sie insbesondere durch die Zellen der CHO (Chinese Hamster Ovary)-Zelllinie gebildet wird.

19. Verfahren zur Expression eines Peptids nach einem der Ansprüche 9 bis 12, **dadurch gekennzeichnet, dass** die Wirtszelle, die aus der Transformation durch einen Vektor resultiert, welcher eine Nukleotidsequenz enthält, die für ein Peptid murinen Ursprungs mit β3-Adrenozeptor-Aktivität codiert, derart kultiviert wird, dass das Peptid produziert wird und das exprimierte Peptid zur Membran transportiert wird, derart, dass die Transmembransequenzen des Rezeptors β3 an der Oberfläche der Membran des transformierten Zellwirts exponiert sind.

20. Untersuchungsmodell für β3-Adrenozeptoren, **dadurch gekennzeichnet, dass** es aus Wirtszellen nach Anspruch 17 oder 18 besteht, d.h. einen murinen β3-Adrenozeptor an der Oberfläche ihrer Membran exprimiert.

21. Verfahren zum Nachweis der Fähigkeit einer Substanz, sich gegenüber einem Peptid nach einem der Ansprüche 9 bis 12 als Ligand zu verhalten, wobei das Verfahren umfasst:
- Inkontaktbringen der Substanz mit einer Wirtszelle, die vorher durch einen Expressionsvektor nach einem der Ansprüche 13 bis 16 transformiert worden war, wobei die Wirtszelle das murine Peptid (muriner Raβ3), ggf. nach geeigneter physikalischer oder chemischer Induzierung, exprimiert und das Inkontaktbringen unter Bedingungen erfolgt, die die Bildung einer Bindung zwischen mindestens einer der spezifischen Stellen und der Substanz, wenn er erfolgt, durchgeführt wird, und
- den Nachweis der eventuellen Bildung eines Komplexes des Typs Ligand-Peptid.

22. Verfahren zur Herstellung von rekombinanten Mäusen, die den β3-Adrenozeptor nicht mehr exprimieren, dadurch gekennzeichnet, dass
- man in der Sequenz der Formel I nach Anspruch 1 eine zweckdienliche Mutation durchführt, die die Expression des Gens hemmt, das für murinen β3-Adrenozeptor codiert;
- man die modifizierte Sequenz I in ein Segment der Genom-DNA der Maus, gebunden an einen geeigneten Marker, derart einführt, dass man eine markierte Sequenz M enthält, die die modifizierte Sequenz der Formel I enthält;
- man die Sequenz M in vitro in die Keimstammszellenlinien des Mausembryo einführt,
- man dann die Stammzellen wieder einer Maus injiziert und
- man nach homologer Rekombination in der Generation F2 rekombinierte Maushomozygoten erhält, die durch die Anwesenheit des Markers erkennbar sind.

23. Verfahren nach Anspruch 22, **dadurch gekennzeichnet, dass** das Segment der Maus-Genom-DNA höchstens 14 Kilobasen umfasst.

24. Verfahren nach Anspruch 23, **dadurch gekennzeichnet, dass**, wenn das Fragment 14 Kilobasen umfasst, es insbesondere die folgenden Restriktionsspaltstellen umfasst: 3 Stellen SphI, 2 Stellen BamHI, 2 Stellen HindIII, 4 Stellen XbaI, 4 Stellen SacI, 1 Stelle KpnI, 1 Stelle SmaI, 2 Stellen BglII, 2 Stellen PvuII, 1 Stelle PstI und 1 Stelle EcoRI.

25. Verfahren nach einem der Ansprüche 22 bis 24, **dadurch gekennzeichnet, dass** die mutierte Sequenz der Formel I zwischen einer BglII-Stelle und einer BamHI-Stelle des Segments des Maus-DNA-Genoms enthalten ist.

26. Verfahren zur Untersuchung der Affinität eines Peptids nach einem der Ansprüche 9 bis 12 für einen oder mehrere bestimmte Liganden, wobei das Verfahren umfasst:
- die Transformation einer geeigneten Wirtszelle durch einen Expressionsvektor nach einem der Ansprüche 13 bis 16;
- die Kultur der transformierten Wirtszelle unter Bedingungen, die die Expression des β3-Rezeptors, der durch die Nukleotidsequenz codiert wird, und den Transfer des exprimierten β3-Rezeptors zur Membran der Zelle ermöglichen, derart, dass die Transmembransequenzen des β3-Rezeptors an der Oberfläche der transformierten Wirtszelle exponiert sind;
- Inkontaktbringen der Zelle mit den bestimmten Liganden; und
- Nachweis einer Reaktion zwischen der transformierten Zelle und den bestimmten Liganden.

27. Kit oder Satz zum Nachweis der eventuellen Affinität eines Liganden für ein Peptid nach einem der Ansprüche 9 bis 12, wobei der Kit umfasst:
- eine Kultur von Wirtszellen, die durch einen Expressionvektor nach einem der Ansprüche 13 bis 16 transformiert sind;
- physikalische oder chemische Mittel zum Einleiten der Expression eines Peptids (muriner β3-Rezeptor oder ein Fragment desselben), das durch eine Nukleotidsequenz nach einem der Ansprüche 1 bis 4 codiert wird, die in einem Vektor enthalten ist, dessen Promotor induzierbar ist;
- ein oder mehrere Vergleichsliganden, die bestimmte Affinitäten zu dem Peptid haben; und
- physikalische oder chemische Mittel zur Charakterisierung der biologischen Aktivität des exprimierten Peptids.

28. Antikörper, die in spezifischer Weise gegen eines der Peptide nach einem der Ansprüche 9 bis 12 gerichtet sind, **dadurch gekennzeichnet, dass** sie weder den β1-Adrenozeptor noch den β2-Adrenozeptor erkennen.

29. Antikörper nach Anspruch 28, **dadurch gekennzeichnet, dass** sie polyklonale Antikörper sind.

30. Antikörper nach Anspruch 28, **dadurch gekennzeichnet, dass** sie monoklonale Antikörper sind.
